# EUROPEAN PATENT SPECIFICATION

(11) **EP 2 083 270 B1**
(45) Date of publication and mention of the grant of the patent: **12.06.2019**
(21) Application number: 08169401.0
(22) Date of filing: 26.03.2004
(51) Int. Cl.: G01N 33/53, G01N 33/68, G01N 33/569, G01N 33/574, G01N 33/541

(54) **A METHOD AND KIT FOR DETECTING THE EARLY ONSET OF RENAL TUBULAR CELL INJURY**
VERFAHREN UND KIT ZUM NACHWEIS DES FRÜHSTADIUMS EINER NIERENTUBULUSZELLENVERLETZUNG
PROCEDE ET TROUSSE PERMETTANT DE DETECTER L'APPARITION PRECOCE DE LESIONS DE CELLULES TUBULAIRES RENALES

(30) Priority: 27.03.2003 US 458143 P; 04.11.2003 US 481596 P
(43) Date of publication of application: 29.07.2009
(62) Divisional of application: 04758356.2
(73) Proprietor: Children's Hospital Medical Center, Cincinnati, Ohio 45229 (US); The Trustees of Columbia University, New York NY 10027 (US)
(72) Inventor: Devarajan, Prased, Cincinnati, OH 45242 (US); Barasch, Jonathan M., New York, NY 10023 (US)
(74) Representative: Høiberg P/S

(56) References cited:
- YAN LI ET AL: "The high molecular weight urinary matrix metalloproteinase (MMP) activity is a complex of gelatinase B/MMP-9 and neutrophil gelatinase-associated lipocalin (NGAL). Modulation of MMP-9 activity by NGAL.MODULATION OF MMP-9 ACTIVITY BY NGAL" JOURNAL OF BIOLOGICAL CHEMISTRY, AMERICAN SOCIETY OF BIOLOCHEMICAL BIOLOGISTS, BIRMINGHAM,, US, vol. 276, no. 40, 5 October 2001 (2001-10-05), pages 37258-37265, XP002226670 ISSN: 0021-9258
- MATTHAEUS T ET AL: "Acute ischemic renal failure induces expression of neutrophil gelatinase-associated lipocalin and matrix metalloproteinase-9 in damaged tubuli" KIDNEY AND BLOOD PRESSURE RESEARCH, vol. 24, no. 4-6, 2001, page 342, XP009065224 & JOINT SCIENTIFIC MEETING OF THE NEPHROLOGY SOCIETY AND THE GERMAN WORKING GROUP FOR CLINICAL NEPHROL; MUNSTER, GERMANY; SEPTEMBER 29-OCTOBER 02, 2001 ISSN: 1420-4096
- MISHRA JAYA ET AL: "Identification of neutrophil gelatinase-associated lipocalin as a novel early urinary biomarker for ischemic renal injury." JOURNAL OF THE AMERICAN SOCIETY OF NEPHROLOGY : JASN. OCT 2003, vol. 14, no. 10, October 2003 (2003-10), pages 2534-2543, XP002377943 ISSN: 1046-6673
- MONIER F ET AL: "Gelatinase isoforms in urine from bladder cancer patients" CLINICA CHIMICA ACTA, ELSEVIER BV, AMSTERDAM, NL, vol. 299, no. 1-2, 1 September 2000 (2000-09-01), pages 11-23, XP002229630 ISSN: 0009-8981
- MOSES M A ET AL: "Increased incidence of matrix metalloproteinases in urine of cancer patients" CANCER RESEARCH, AMERICAN ASSOCIATION FOR CANCER RESEARCH, BALTIMORE, MD., US, vol. 58, no. 7, 1 April 1998 (1998-04-01), pages 1395-1399, XP002226669 ISSN: 0008-5472
- MATTHAEUS T ET AL: "CO-REGULATION OF NEUTROPHIL GELATINASE-ASSOCIATED LIPOCALIN AND MATRIX METALLOPROTEINASE-9 INTHE POSTISCHEMIC RAT KIDNEY" JOURNAL OF THE AMERICAN SOCIETY OF NEPHROLOGY, WILLIAMS AND WILKINS, BALTIMORE, MD, US, vol. 12, 10 October 2001 (2001-10-10), page 787A,ABSTR.NR.#4112, XP009065221 ISSN: 1046-6673
- BLASER ET AL: "A sandwich enzyme immunoassay for the determination of neutrophil lipocalm in body fluids" CLINICA CHIMICA ACTA, ELSEVIER BV, AMSTERDAM, NL, vol. 235, no. 2, 31 March 1995 (1995-03-31), pages 137-145, XP002976750 ISSN: 0009-8981
- OHLSSON S ET AL: "Increased circulating levels of proteinase 3 in patients with anti-neutrophilic cytoplasmic autoantibodies-associated systemic vasculitis in remission" CLINICAL AND EXPERIMENTAL IMMUNOLOGY, WILEY-BLACKWELL PUBLISHING LTD, GB, vol. 131, no. 3, 1 March 2003 (2003-03-01), pages 528-535, XP002467651 ISSN: 0009-9104
- ELNEIHOUM A M ET AL: "Leukocyte activation detected by increased plasma levels of inflammatory mediators in patients with ischemic cerebrovascular diseases" STROKE, LIPPINCOTT WILLIAMS & WILKINS, US, vol. 27, 1 January 1996 (1996-01-01), pages 1734-1738, XP002982102 ISSN: 0039-2499

## Description

### BACKGROUND

Acute renal failure (ARF) secondary to a renal tubular cell injury, including an ischemic injury or a nephrotoxic injury remains a common and potentially devastating problem in clinical medicine and nephrology, with a persistently high rate of mortality and morbidity despite significant advances in supportive care. Pioneering studies over several decades have illuminated the roles of persistent vasoconstriction, tubular obstruction, cellular structural and metabolic alterations, and the inflammatory response in the pathogenesis of ARF. While these studies have suggested possible therapeutic approaches in animal models, translational research efforts in humans have yielded disappointing results. The reasons for this may include the multifaceted response of the kidney to ischemic injury and nephrotoxins, and a paucity of early biomarkers for ARF with a resultant delay in initiating therapy.

An individual is considered to have acute renal failure when the patient's serum creatinine value either: (1) increased by at least 0.5 mg/dL when the baseline serum creatinine level was less than 2.0 mg/dL; (2) increased by at least 1.5 mg/dL when the baseline serum creatinine level was greater than or equal to 2.0 mg/dL; or (3) increased by at least 0.5 mg/dL, regardless of the baseline serum creatinine level, as a consequence of exposure to radiographic agents.

It is believed that introduction of therapy early in the disease process will reduce the mortality rate associated with ARF and shorten the time for treatment of various types of renal tubular cell injuries, including, but not limited to, ischemic and nephrotoxic renal injuries. The identification of a reliable, early biomarker for a renal tubular cell injury would be useful to facilitate early therapeutic intervention, and help guide pharmaceutical development by providing an indicator of nephrotoxicity.

The traditional laboratory approach for detection of renal disease involved determining the serum creatinine, blood urea nitrogen, creatinine clearance, urinary electrolytes, microscopic examination of the urine sediment, and radiological studies. These indicators are not only insensitive and nonspecific, but also do not allow for early detection of the disease. Indeed, while a rise in serum creatinine is widely considered as the "gold standard" for the detection of ARF, it is now clear that as much as 50% of the kidney function may already be lost by the time the serum creatinine changes.

A few urinary biomarkers for ischemic renal injury have been earlier described, including kidney injury molecule-1 (KIM-1) and cysteine rich protein 61 (Cyról). KIM-1 is a putative adhesion molecule involved in renal regeneration. In a rat model of ischemia-reperfusion injury, KIM-1 was found to be upregulated 24-48 hours after the initial insult, rendering it a reliable but somewhat late marker of tubular cell damage. Recent studies have shown that KIM-1 can be detected in the kidney biopsy and urine of patients with ischemic acute tubular necrosis. However, this detection was documented in patients with established ischemic renal damage, late in the course of the illness. The utility of urinary KIM-1 measurement for the detection of early ARF or subclinical renal injury has thus far not been validated.

The protein Cyr61 was found to be a secreted cysteine-rich protein that is detectable in the urine 3-6 hours after ischemic renal injury in animal models. However, this detection required a bioaffmity purification and concentration step with heparin-sepharose beads, followed by a Western blotting protocol. Even after bioaffmity purification several nonspecific cross-reacting peptides were apparent. Thus, the detection of Cyr61 in the urine is problematic with respect to specificity as well as the cumbersome nature of the procedure.

Yan li et al. disclose in "The high molecular weight urinary matrix metalloproteinase (MMP) activity is a complex of gelatinase B/MMP-9 and neutrophil gelatinase-associated lipocalin (NGAL). Modulation of MMP-9 activity by NGAL" published in Journal of Biological Chemistry 2001; 276;40: 37258-65 that NGAL is present in the urine of patients with renal and bladder cancer. Matthaeus et al. disclose in "Acute ischemic renal failure induces expression of neutrophil gelatinase-associated lipocalin and matrix metalloproteinase-9 in damaged tubuli" published in Kidney and Blood Pressure Research 2001; 24: 342 and in "Co-regulation of neutrophil gelatinase-associated lipocalin and matrix metalloproteinase-9 in the postischemic rat kidney" published in Journal of the American Society of Nephrology 2001; 12: 787A that NGAL protein expression has been detected by Western blot in injured proximal tubuli after acute ischemic renal failure. Blaser et al disclose in "A sandwich enzyme immunoassay for the determination of neutrophil lipocalin in body fluids" published in Clinica Chimica Acta 1995; 235;2: 137-145 that NGAL is detectable in urine of healthy subjects. Therefore, there remains an urgent need to identify improved biomarkers for early ischemic and nephrotoxic renal injuries.

### SUMMARY

The present disclosure relates to a method for the detection of a renal tubular cell injury in a mammal, comprising the steps of: 1) obtaining a urine sample from a mammalian subject; 2) contacting the urine sample with an antibody for a renal tubular cell injury biomarker, the renal tubular cell injury biomarker comprising NGAL, to allow formation of a complex of the antibody and the renal tubular cell injury biomarker; and 3) detecting the antibody-biomarker complex.

The present disclosure relates to a method of monitoring the effectiveness of a treatment for renal tubular cell injury comprising the steps of: 1) providing a treatment to a mammalian subject experiencing ischemic renal injury; 2) obtaining at least one post-treatment urine sample from the subject; and 3) detecting for the presence of a biomarker for renal tubular cell injury in the post-treatment urine sample.

The present disclosure further relates to a kit for use in detecting the presence of an immediate or early onset biomarker for renal tubular cell injury in the urinary fluid of a subject, comprising: 1) a means for acquiring a quantity of a urine sample; 2) a media having affixed thereto a capture antibody capable of complexing with an renal tubular cell injury biomarker, the biomarker being NGAL; and 3) an assay for the detection of a complex of the renal tubular cell injury biomarker and the capture antibody.

The present disclosure also relates to a competitive enzyme linked immunosorbent assay (ELISA) kit for determining the renal tubular cell injury status of a mammalian subject, comprising a first antibody specific to a renal tubular cell injury biomarker to detect its presence in a urine sample of the subject.

The present disclosure further relates to a method of identifying the extent of a renal tubular cell injury caused by an event, comprising: 1) obtaining at least one urine sample from a mammalian subject; 2) detecting in the urine sample the presence of a biomarker for renal tubular cell injury; and 3) determining the extent of renal tubular cell injury based on the time for onset of the presence of IRI biomarker in the urine sample, relative to the time of the event.

The present present disclosure further relates to a method for the detection of a renal tubular cell injury in a mammal, comprising the steps of: 1) obtaining a urine sample comprising up to 1 milliliter of the first urine from a mammalian subject following a suspected renal tubular cell injury; 2) contacting the urine sample with an antibody for a biomarker for renal tubular cell injury, to allow formation of a complex of the antibody and the biomarker; and 3) detecting the antibody-biomarker complex.

A preferred renal tubular cell injury biomarker is NGAL.

### BRIEF DESCRIPTION OF THE DRAWINGS

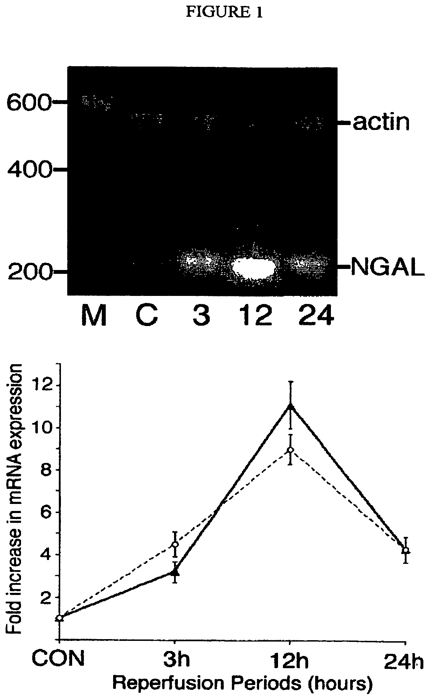

Figure 1 shows induction of mouse kidney NGAL mRNA following ischemia. Top panel shows a representative RT-PCR with primers for mouse actin and NGAL, using RNA extracted from kidneys of control (C) mice or after various reperfusion periods as shown (hours). Lane M contains a molecular weight standard marker. Bottom panel shows the fold increase in NGAL mRNA expression at various time points from control (CON). Values obtained by microarray (solid line) vs RT-PCR (dotted line) are means +/- SD from at least 3 experiments.

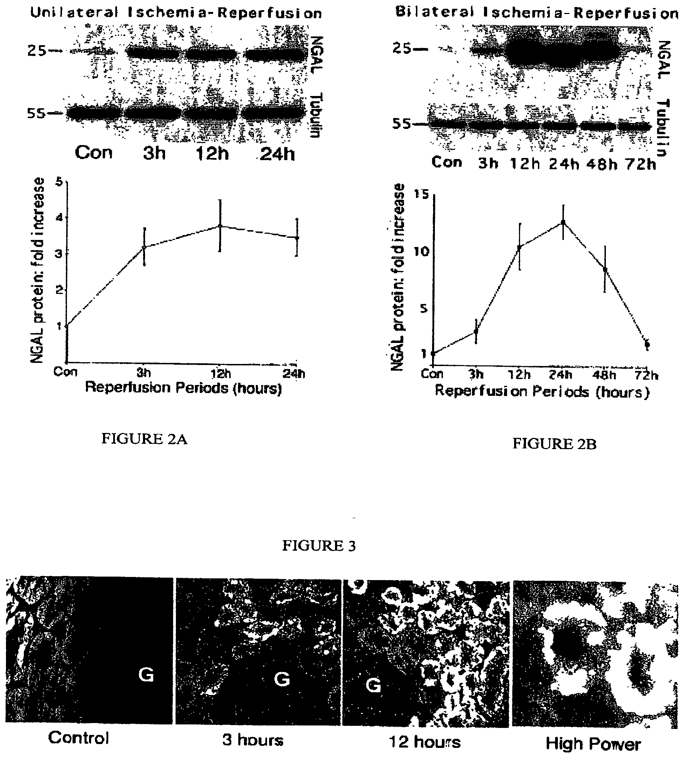

Figure 2A shows induction of mouse kidney NGAL protein following unilateral ischemia. Top panel shows a representative Western blot with whole kidney samples obtained from control (Con) mice or after reperfusion periods as shown (hours), probed with a polyclonal antibody to NGAL or a monoclonal antibody to tubulin (to demonstrate equal protein loading). Molecular weight markers are to the left. Bottom panel shows the fold increase in NGAL protein expression at various time points from control (CON). Values obtained by densitometry are means +/- SD from at least 3 experiments.
Figure 2B shows induction of mouse kidney NGAL protein following bilateral ischemia. Top panel shows a representative Western blot with whole kidney samples obtained from control (Con) mice or after reperfusion periods as shown (hours), probed with a polyclonal antibody to NGAL or a monoclonal antibody to tubulin (to demonstrate equal protein loading). Molecular weight markers are to the left. Bottom panel shows the fold increase in NGAL protein expression at various time points from control (CON). Values obtained by densitometry are means +/- SD from at least 3 experiments.
Figure 3 shows induction of mouse kidney NGAL protein following ischemia. Representative immunohistochemistry results on frozen sections of mouse kidneys obtained from control mice or after varying periods of reflow as shown (hours), probed with a polyclonal antibody to NGAL. "G" denotes a glomerulus. The panel on the extreme right is a 100X magnification, and the other panels are at 20X.

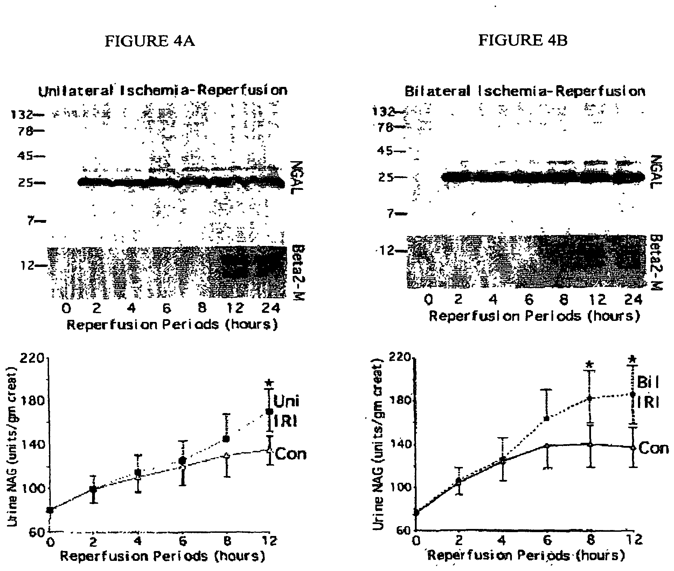

Figure 4A shows early detection of NGAL protein in the urine in mice with unilateral ischemic ARF. Representative Western blot of unprocessed urine samples (1-2 µl per lane, normalized for creatinine content) obtained at reperfusion periods as shown (hours), following unilateral renal artery clamping. Molecular weight markers are shown on the right. Blots were probed with NGAL (top) or β2-microglobulin (Beta2-M) (middle). Urinary N-acetyl- β-D-glucosaminidase (NAG) determinations at various reperfusion periods as indicated, from five animals for five animals. Values are means +/- SD. *P < 0.05 versus control at each time period, ANOVA.
Figure 4B shows early detection of NGAL protein in the urine in mice with bilateral ischemic ARF. Representative Western blot of unprocessed urine samples (1-2 µl per lane, normalized for creatinine content) obtained at reperfusion periods as shown (hours), following bilateral renal artery clamping. Molecular weight markers are shown on the right. Blots were probed with NGAL (top) or β2-microglobulin (Beta2-M) (middle). Urinary N-acetyl- β-D-glucosaminidase (NAG) determinations at various reperfusion periods as indicated, from five animals for eight animals. Values are means +/- SD. *P < 0.05 versus control at each time period, ANOVA.

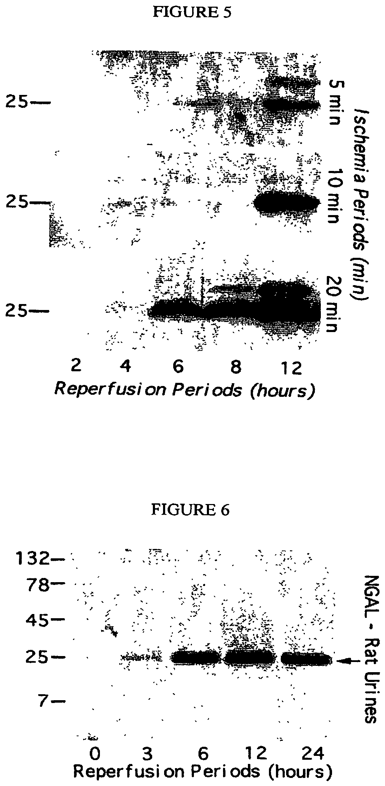

Figure 5 shows detection of NGAL protein in the urine from mice with subclinical renal ischemia. Representative Western blot of unprocessed urine samples (1-2 µl per lane, normalized for creatinine content) obtained at reperfusion periods as shown (hours), following 5, 10, or 20 min of bilateral renal artery clamping. Molecular weight markers are shown on the left. These animals displayed normal serum creatinines at 24 h of reflow.
Figure 6 shows early detection of NGAL protein in the urine in rats with ischemic ARF, Representative Western blot of unprocessed urine samples (1-2 µl per lane, normalized for creatinine content) obtained at reperfusion periods as shown (hours), following 30 min of bilateral renal artery clamping in rats. Molecular weight markers are shown on the left. These animals displayed a significant increase in serum creatinine at 24 h of reflow.

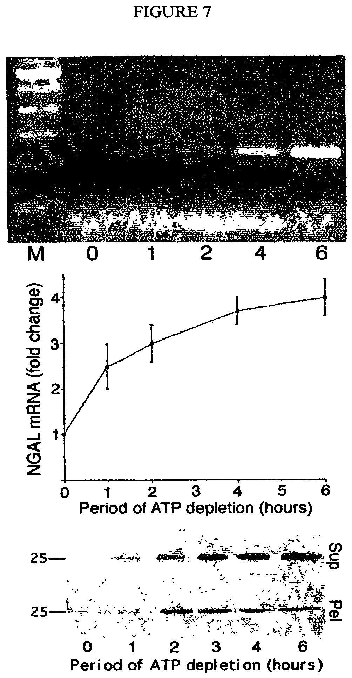

Figure 7 shows induction of NGAL mRNA following ischemia in vitro. Top panel shows a representative RT-PCR with primers for human NGAL, using RNA extracted from renal proximal tubular epithelial cells (RPTEC) after various periods of partial ATP depletion as shown (hours). Lane M contains a 100 bp DNA ladder. The middle panel shows the fold increase in NGAL mRNA expression at various time points from control (0), normalized for glyceeraldehyde-3-ohosphate dehydrogenase (GAPDH) expression. Values shown are means +/- SD from at least 3 experiments at each point. The bottom panel shows a representative Western blot (of three separate experiments) with RPTEC samples after various periods of partial ATP depletion as shown, obtained from equal amounts of cell pallets (Pel) or the culture medium (Sup), probed with a polyclonal antibody to NGAL. Molecular weight markers are to the left.

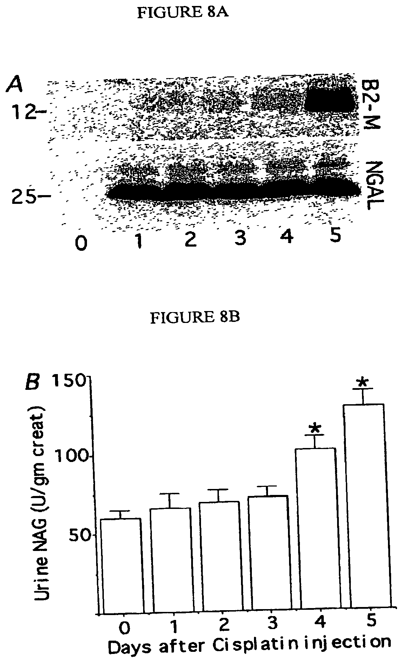

Figure 8A shows early detection of NGAL protein in the urine was detected in mice with cisplatin-induced injury. Representative Western blots on unprocessed urine samples (1-2 µl per lane, normalized for creatinine content) obtained at days as shown following cisplatin administration, probed with antibody for β-2-microglobulin (top panel) and NGAL (middle panel). Molecular weight markers are shown on the left.
Figure 8B shows urinary NAG determinations at various days after cisplatin administration (n=4) in Figure 8A. Values are means +/- SD. *P < 0.05 versus day 0.

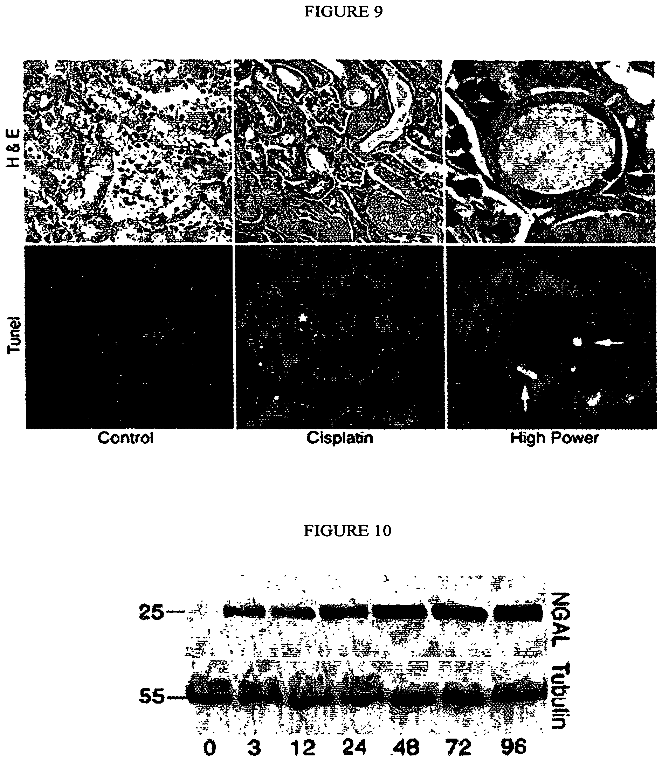

Figure 9 shows that cisplatin administration results in tubule cell necrosis and apoptosis. Hematoxylin-eosin stain showed tubular dilatation, luminal debris, and flattened epithelium in cisplatin-treated kidneys (top center panel). At high power, a tubule marked with an asterisk displayed condensed intensely-stained nuclei (arrow), indicative of apoptosis (top right panel). TUNEL staining showing TUNEL-positive nuclei in cisplatin-treated kidneys (bottom center panel). At high power, the tubule indicated by an asterisk displayed condensed, fragmented nuclei (arrow) characteristic of apoptosis (bottom right panel). Panels labeled High Power are at 100X magnification, and the others are at 20X. Results in control mice are shown in top and bottom left panels.
Figure 10 shows that cisplatin administration results in rapid induction of kidney NGAL. Representative Western blots of kidney lysates from mice treated with intraperitoneal cisplatin (20 µg/kg) and obtained at various time points as indicated (hours), probed with a polyclonal antibody to NGAL or a monoclonal antibody to tubulin. Molecular weight markers are to the left.

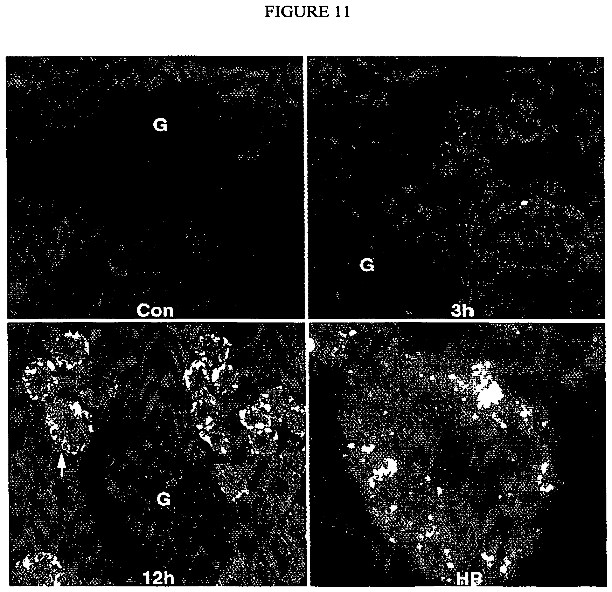

Figure 11 shows that cisplatin administration results in rapid induction of NGAL in tubule epithelial cells. Representative immunohistochemistry results on frozen kidney sections from mice treated with intraperitoneal cisplatin (20 µg/kg) and obtained at various time points as indicated (hours), probed with a polyclonal antibody to NGAL. G, glomerulus. Panel labeled HP is at 100X magnification, and the others are at 20X.

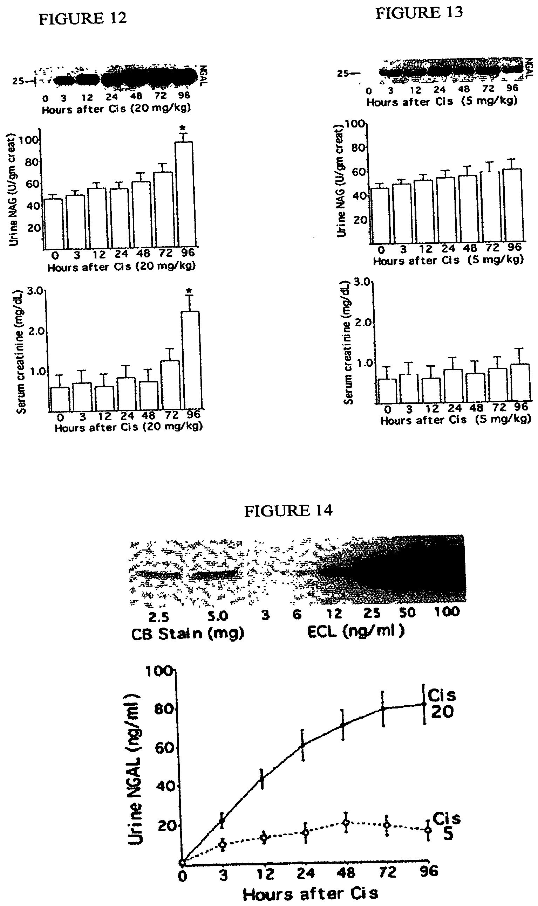

Figure 12 shows that administration of 20 µg/kg cisplatin results in rapid appearance of NGAL in the urine. Representative Western blot (upper panel) of unprocessed urine samples (3-5 µl/lane, normalized for creatinine content) obtained before or at various time points following cisplatin injections as shown. The same urine samples were analyzed for NAG excretion (center panel), and serum from the same animals subjected to creatinine measurement (bottom panel). *P<0.05 versus control.
Figure 13 shows that administration of 5 µg/kg cisplatin results in rapid appearance of NGAL in the urine. Representative Western blot (upper panel) of unprocessed urine samples (3-5 µl/lane, normalized for creatinine content) obtained before or at various time points following cisplatin injections as shown. The same urine samples were analyzed for NAG excretion (center panel), and serum from the same animals subjected to creatinine measurement (bottom panel). *P<0.05 versus control.
Figure 14 shows quantitation of urinary NGAL following cisplatin. Coomassie Blue (CB) staining (top left panel) and Enhanced Chemiluminescence (ECL) analysis of known quantities of recombinant purified NGAL (top right panel). Quantitation of urinary NGAL excretion at various time points following cisplatin 20 µg/kg or 5 µg/kg, determined by densitometric analysis of Western blots and comparisons with Western blots of defined standards of purified NGAL performed under identical conditions.

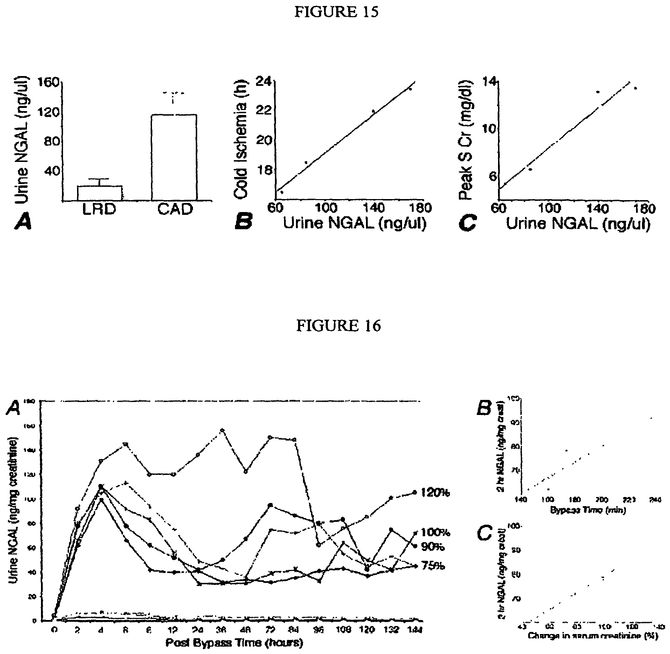

Figure 15 shows in panel A the measurement of urine NGAL in patients with cadaveric kidney transplants (CAD, n=4) versus living related donor transplants (LRD, n = 6) (p < 0.005). Panel B shows a correlation between cold ischemia time and urinary NGAL in CAD (p < 0.001, r = 0.98, Spearman analysis). Panel C shows a correlation between peak serum creatinine and urinary NGAL in CAD (p < 0.001, r =0.96, Spearman analysis).
Figure 16 shows in panel A the results of serial measurements of urinary NGAL in patients following open heart surgery, plotted against post bypass time in hours (n=15). Panel B shows a correlation between bypass time and the 2 hour urinary NGAL in patients who developed ARF (n = 5) (p< 0.01, r = 0.76, Spearman analysis). Panel C shows a correlation between changes in serum creatinine and the 2 hour urinary NGAL in patients who developed ARF (p < 0.01, r = 0.66, Spearman analysis).

### DETAILED DESCRIPTION

The invention is defined by the appended claims. Any embodiment not falling under the scope of the appended claims does not form part of the invention. Throughout this application, various publications and unpublished manuscripts are referred to within parentheses. Full bibliographic citation for these references can be found at the end of this application, preceding the claims.

The present disclosure provides a method and kit for assaying the presence of a renal tubular cell injury biomarker present in the urine of a subject at the early onset of renal tubular cell injury. Early detection of the onset of the injury can reduce the time for treatment of the injury, and can reduce the risk of developing clinical acute renal failure (ARF). The renal tubular cell injury can include, but is not limited to, ischemic renal injury (IRI) or nephrotoxic renal injury (NRI).

A simple point-of-care kit that uses principles similar to the widely-used urine pregnancy testing kits, for the rapid detection of urinary NGAL at the bedside will allow the clinician to rapidly diagnose ARF, and to rapidly institute proven and effective therapeutic and preventive measures. The use of the kit can represent the standard of care for all patients who are at risk of developing ARF, including use in cardiac surgery, kidney transplantation, stroke, trauma, sepsis, dehydration, and nephrotoxins (antibiotics, anti-inflammatory agents, radio-contrast agents, and chemotherapeutic agents). In current clinical practice, when ARF occurs in the setting of these predisposing conditions, the diagnosis is very delayed, and the associated mortality and morbidity unacceptably high. Ironically, even tragically, effective preventive and therapeutic measures are widely available, but almost never administered in a timely manner due to the lack of early biomarkers of ARF. It is anticipated that multiple serial measurements of NGAL will be become indispensable not only for diagnosing and quantifying the initial kidney injury, but also for following the response to early treatment, and for predicting long term outcome.

The biomarker for renal tubular cell injury (which will also be referred to as RTCI biomarker) can be an immediate RTCI biomarker, such as NGAL, which can appear in the urine within 2 hours of the onset of renal tubular cell injury. An immediate RTCI biomarker can, as in the case of NGAL, be present in the first urine output of a subject immediately after the onset of renal tubular cell injury. The RTCI biomarker can also be an early-onset RTCI biomarker that can appear within the first 24 hours of the onset of renal tubular cell injury. As such, NGAL is also an example of an early-onset RTCI biomarker.

An effective RTCI biomarker is typically a secreted protein, whereby it can be excreted by the kidney into the urine. An effective RTCI biomarker is also typically a protease-resistant protein, such as NGAL. Nevertheless, an RTCI biomarker can also be a protease-sensitive protein, so long as stable fragments of the protein can be detected in the urine, such as by antibodies as described hereinafter for NGAL.

The RTCI biomarker can be an ischemic renal injury biomarker (IRI biomarker), a nephrotoxic renal injury biomarker (NRI biomarker), or a mixture thereof. NGAL is an example of both an IRI biomarker and an NRI biomarker.

The method of the present disclosure can be used to detect the onset of renal tubular cell injury, and to monitor the treatment thereof, for a wide variety of events that can include all varieties of diminished blood supply to the kidneys, impaired heart function, surgical procedures, patients in intensive care units, and the administration of pharmaceuticals, radiocontrast dyes, or other medicament substances to a subject. The renal tubular cell injury can be an ischemic renal injury, a nephrotoxic renal injury, or other injury that affects the tubular cells of the kidney. The event can include administration or ingestion of a large and wide variety of nephrotoxins, including, but not limited to cancer chemotherapy (cisplatin, cyclophosphamide, isosfamide, methotrexate), antibiotics (gentamicin, vancomycin, tobramycin), antifungal agents (amphotericin), anti-inflammatory agents (NSAIDs), immunosuppressants (cyclosporine, tacrolimus), and radiocontrast agents. The method can be used to evaluate the nephrotoxisity of both newly-developed and well-known compounds.

The present disclosure also provides a method and a kit for assessing the extent of renal injury based on a proportional relationship between the extent of injury, which can range from the very onset of renal tubular cell injury, to clinical ARF, with the quantity of NGAL present in the urine passing from the subject. The present disclosure provides a means for a clinician to estimate the degree of renal injury at an initial assessment, and to monitor the change in status of the injury (worsening, improving, or remaining the same) based on the detected amount of NGAL in the urine.

Typically, the clinician would establish a protocol of collecting and analyzing a quantity of fresh urine sample from the patient at selected intervals. Typically the sample is obtained intermittently during a prescribed period. The period of time between intermittent sampling may be dictated by the condition of the subject, and can range from a sample each 24 hours to a sample taken continuously, more typically from each 4 hours to each 30 minutes.

Using the methods and techniques described herein, both a qualitative level of the RTCI biomarker present in the urine can be analyzed and estimated, and a quantitative level of RTCI biomarker present in the urine can be analyzed and measured. The clinician would select the qualitative method, the quantitative method, or both, depending upon the status of the patient. Typically, the quantity of urine to be collected is less than 1 milliliter, and more typically less than 10 µl. A typical sample can range from about 1 µl to about 1 ml. Typically the larger quantities of urine sample (about 1 ml) are used for quantitative assays. Typically, these small amounts of urine are easily and readily available from clinical subjects who are either prone to developing ARF, or have developed ARF.

Once an indication of renal tubular cell injury or acute renal failure has been detected, and intervention and treatment of the disease or condition has commenced, the clinician can employ the method and kit of the present disclosure to monitor the progress of the treatment or intervention. Typically, one or more subsequent post-treatment urine samples will be taken and analyzed for the presence of the RTCI biomarker as the treatment of the renal injury commences and continues. The treatment is continued until the presence of the RTCI biomarker in subsequent post-treatment urine samples is not detected. As the treatment and intervention ameliorate the condition, the expression of RTCI biomarker, and its presence in the urine, will be correspondingly reduced. The degree of amelioration will be expressed by a correspondingly reduced level of RTCI biomarker, such as NGAL, detected in a sample. As the renal injury nears complete healing, the method can be used to detect the complete absence of the RTCI biomarker, signaling the completion of the course of treatment.

Both monoclonal and polyclonal antibodies that bind an RTCI biomarker are useful in the methods and kits of the present disclosure. The antibodies can be prepared by methods known in the art. Monoclonal antibodies for a preferred RTCI biomarker, NGAL, are described, for example, in "Characterization of two ELISAs for NGAL, a newly described lipocalin in human neutrophils", Lars Kjeldsen et al., (1996) Journal of Immunological Methods, Vol. 198, 155-16. Examples of monoclonal antibodies for NGAL can be obtained from the Antibody Shop, Copenhagen, Denmark, as HYB-211-01, HYB-211-02, and NYB-211-05. Typically, HYB-211-01 and HYB-211-02 can be used with NGAL in both its reduced and unreduced forms. An example of a polyclonal antibody for NGAL is described in "An Iron Delivery Pathway Mediated by a Lipocalin", Jim Yang et al., Molecular Cell, (2002), Vol. 10, 1045-1056. To prepare this polyclonal antibody, rabbits were immunized with recombinant gel-filtered NGAL protein. Sera were incubated with GST-Sepharose 4B beads to remove contaminants, yielding the polyclonal antibodies in serum, as described by the applicants in Jun Yang et al., Molecular Cell (2002).

Typically, the step of detecting the complex of the capture antibody and the RTCI biomarker comprises contacting the complex with a second antibody for detecting the biomarker.

The method for detecting the complex of the RTCI biomarker and the primary antibody comprises the steps of: separating any unbound material of the urine sample from the capture antibody-biomarker complex; contacting the capture antibody-biomarker complex with a second antibody for detecting the RTCI biomarker, to allow formation of a complex between the RTCI biomarker and the second antibody; separating any unbound second antibody from the RTCI biomarker-second antibody complex; and detecting the second antibody of the RTCI biomarker-second antibody complex .

A kit for use in the method typically comprises a media having affixed thereto the capture antibody, whereby the urine sample is contacted with the media to expose the capture antibody to NGAL contained in the sample. The kit includes an acquiring means that can comprise an implement, such as a spatula or a simple stick, having a surface comprising the media. The acquiring means can also comprise a container for accepting the urine sample, where the container has a urine-contacting surface that comprises the media. In another typical embodiment, the assay for detecting the complex of the RTCI biomarker and the antibody can comprise an ELISA, and can be used to quantitate the amount of NGAL in a urine sample. In an alternative embodiment, the acquiring means can comprise an implement comprising a cassette containing the media.

Early detection of the RTCI biomarker can provide an indication of the presence of the protein in a urine sample in a short period of time. Generally, a method and a kit of the present disclosure can detect the RTCI biomarker in a sample of urine within four hours, more typically within two hours, and most typically within one hour, following renal tubular cell injury. Preferably, the RTCI biomarker can be detected within about 30 minutes following renal tubular cell injury.

A method and kit of the present disclosure for detecting the RTCI biomarker can be made by adapting the methods and kits known in the art for the rapid detection of other proteins and ligands in a biological sample. Examples of methods and kits that can be adapted to the present disclosure are described in US Patent 5,656,503, issued to May et al. on August 12, 1997, US Patent 6,500,627, issued to O'Conner et al. on December 31, 2002, US Patent 4,870,007, issued to Smith-Lewis on September 26, 1989, US Patent 5,273,743, issued to Ahlem et al. on December 28, 1993, and US Patent 4,632,901, issued to Valkers et al. on December 30, 1986.

A rapid one-step method of detecting the RTCI biomarker can reduce the time for detecting the renal tubular cell injury. A typical method can comprise the steps of: obtaining a urine sample suspected of containing the RTCI biomarker; mixing a portion of the sample with a detecting antibody which specifically binds to the RTCI biomarker, so as to initiate the binding the detecting antibody to the RTCI biomarker in the sample; contacting the mixture of sample and detecting antibody with an immobilized capture antibody which specifically binds to the RTCI biomarker, which capture antibody does not cross-react with the detecting antibody, so as to bind the detecting antibody to the RTCI biomarker, and the RTCI biomarker to the capture antibody, to form a detectable complex; removing unbound detecting antibody and any unbound sample from the complex; and detecting the detecting antibody of the complex. The detectable antibody can be labeled with a detectable marker, such as a radioactive label, enzyme, biological dye, magnetic bead, or biotin, as is well known in the art.

To identify potential genes and their proteins that may accompany and mark the earliest onset of renal tubular cell injuries, such as ischemic and nephrotoxic renal injuries, a cDNA microarray assay can be used to detect which of a large number of potential gene targets are markedly upregulated. Utilizing this screening technique, neutrophil gelatinase-associated lipocalin (NGAL) was identified as a gene whose expression is upregulated more than 10 fold within the first few hours following an ischemic renal injury in a mouse model.

NGAL belongs to the lipocalin superfamily of over 20 structurally related secreted proteins that are thought to transport a variety of ligands within a β-barreled calyx. Human NGAL was originally identified as a 25 kDa protein covalently bound to gelatinase from human neutrophils, where it represents one of the neutrophil secondary granule proteins. Molecular cloning studies have revealed human NGAL to be similar to the mouse 24p3 gene first identified in primary cultures of mouse kidneys that were induced to proliferate.

NGAL is expressed at very low levels in other human tissues, including kidney, trachea, lungs, stomach, and colon. NGAL expression is markedly induced in stimulated epifhelia. For example, it is upregulated in colonic epithelial cells in areas of inflammation or neoplasia, but is absent from intervening uninvolved areas or within metastatic lesions. NGAL concentrations are elevated in the serum of patients with acute bacterial infections, the sputum of subjects with asthma or chronic obstructive pulmonary disease, and the bronchial fluid from the emphysematous lung. In all these cases, NGAL induction is postulated to be the result of interactions between inflammatory cells and the epithelial lining, with upregulation of NGAL expression being evident in both neutrophils and the epithelium.

It is believed that the detected NGAL induction represents a novel intrinsic response of the kidney proximal tubule cells to renal tubular cell injuries, including both ischemic and nephrotoxic injuries, and is not derived merely from activated neutrophils. First, the response is rapid, with NGAL appearing in the urine within 2 hours of the injury with the very first urine output following renal artery occlusion, while renal neutrophil accumulation in this model of ischemic ARF is usually first noted at 4 hours after injury. Second, the temporal patterns of NGAL induction and neutrophil accumulation are divergent. NGAL mRNA and protein expression was maximally noted at 12 hours of reflow, whereas neutrophil accumulation peaks at 24 hours by which time NGAL expression has significantly declined. Third, no NGAL-expressing neutrophils were detectable by immunofluorescence in the kidney samples examined (Figure 3). Fourth, NGAL mRNA and protein induction was documented to occur in cultured human proximal tubule cells following in vitro ischemia, with NGAL secreted into the culture medium within 1 hour of ATP depletion, in a system where neutrophils are absolutely absent. Nevertheless, some contribution from infiltrating neutrophils to the observed NGAL upregulation may have occurred. It is possible that upregulation of NGAL in renal tubule cells may be induced by local release of cytokines from neutrophils trapped in the microcirculation early after ischemic injury.

An adequate explanation for the induction of NGAL by stimulated epif elia has been lacking, and whether NGAL is protective or proximate to injury or even an im ocent bystander remains unclear. Recent evidence suggests that, at least in a subset of cell types, NGAL may represent a pro-apoptotic molecule. In the mouse pro-B lymphocytic cell line, cytokine withdrawal resulted in a marked induction of NGAL as well as onset of apoptosis. Purified NGAL produced the same pro-apoptotic response as cytokine deprivation, including activation of Bax, suggesting that NGAL is proximate to programmed cell death. NGAL has also been linked to apoptosis in reproductive tissues. Epithelial cells of the involuting mammary gland and uterus express high levels of NGAL, temporally coinciding with a period of maximal apoptosis. It is likely that NGAL regulates a subset of cell populations by inducing apoptosis. Stimulated epifhelia may upregulate NGAL in order to induce apoptosis of infiltrating neutrophils, thereby allowing the resident cells to survive the ravages of the inflammatory response. Alternatively, epithelial cells may utilize this mechanism to regulate their own demise. However, it is interesting to note that induction of NGAL following renal ischemia-reperfusion injury occurs predominantly in the proximal tubule cells, and apoptosis under the same circumstances is primarily a distal tubule cell phenomenon.

Other recent studies have revealed that NGAL enhances the epithelial phenotype. NGAL is expressed by the penetrating rat ureteric bud, and triggers nephrogenesis by stimulating the conversion of mesenchymal cells into kidney epifhelia. Another lipocalin, glycodelin, has been shown to induce an epithelial phenotype when expressed in human breast carcinoma cells. These findings are especially pertinent to the mature kidney, in which one of the well-documented responses to ischemic injury is the remarkable appearance of dedifferentiated epithelial cells lining the proximal tubules. An important aspect of renal regeneration and repair after ischemic injury involves the reacquisition of the epithelial phenotype, a process that recapitulates several aspects of normal development. This suggests that NGAL may be expressed by the damaged tubule in order to induce re-epithelialization. Support for this notion derives from the recent identification of NGAL as an iron transporting protein that is complementary to transferrin during nephrogenesis. It is well known that the delivery of iron into cells is crucial for cell growth and development, and this is presumably critical to postischemic renal regeneration just as it is during ontogeny. Since NGAL appears to bind and transport iron, it is also likely that NGAL may serve as a sink for iron that is shed from damaged proximal tubule epithelial cells. Because it has been observed that NGAL can be endocytosed by the proximal tubule, the protein could potentially recycle iron into viable cells. This might stimulate growth and development, as well as remove iron, a reactive molecule, from the site of tissue injury, thereby limiting iron-mediated cytotoxicity.

NGAL is a novel urinary biomarker for cisplatin-induced nephrotoxic renal injury that is more sensitive than previously described biomarkers. One example is kidney injury molecule- 1 or KIM-1, a putative adhesion molecule involved in renal regeneration. In a rat model of cisplatin nephrotoxicity, KIM-1 was qualitatively detectable 24-48 hours after the initial insult, rendering it a somewhat late marker of tubular cell damage. In contrast, NGAL is readily and quantitatively detected within 3 hours following cisplatin administration in doses known to result in renal failure. In addition, urinary NGAL detection precedes the appearance of other markers in the urine such as NAG. Appearance of NGAL in the urine also precedes the increase in serum creatinine that is widely used to diagnose nephrotoxic renal failure.

Urinary NGAL is evident even after mild "sub-clinical" doses of cisplatin, in spite of normal serum creatinine levels. Thus, the present disclosure has important implications for the clinical management of patients on cisplatin therapy. The efficacy of cisplatin is dose dependent, but the occurrence of nephrotoxicity frequently hinders the use of higher doses to maximize its antineoplastic potential. Nephrotoxicity following cisplatin treatment is common and may manifest after a single dose with acute renal failure. Although several therapeutic maneuvers have proven to be efficacious in the treatment of cisplatin-induced nephrotoxicity in animals, successful human experiences have remained largely anecdotal. One reason for this may be the lack of early markers for nephrotoxic acute renal failure, and hence a delay in initiating therapy. In current clinical practice, acute renal injury is typically diagnosed by measuring serum creatinine. However, it is well known that creatinine is an unreliable and delayed indicator during acute changes in kidney function. First, serum creatinine concentrations may not change until about 50% of kidney function has already been lost. Second, serum creatinine does not accurately depict kidney function until a steady state has been reached, which may require several days. Thus, the use of serum creatinine measurements impairs the ability to both detect and quantify renal damage during the early phases of renal injury. However, animal studies have suggested that while nephrotoxic acute renal failure can be prevented and/or treated, there is a narrow "window of opportunity" to accomplish this, and treatment must be instituted very early after the initiating insult. The lack of early biomarkers of renal injury has impaired the ability of clinicians to initiate potentially effective therapies in a timely manner. The use of NGAL in an assay system would also be of value for testing existing or emerging therapeutic or preventive interventions, and for the early evaluation of the nephrotoxic potential of other pharmaceutical agents. NGAL detection is a novel, non-invasive, early urinary biomarker for cisplatin-induced kidney damage. Early detection may enable clinicians to administer timely therapeutic interventions, and to institute maneuvers that prevent progression to overt nephrotoxic renal failure.

It has been found that NGAL was easily and rapidly detected as relatively clean immunoreactive peptides in Western blots with as little as 1 µl of the very first unprocessed urine output following renal ischemia in both mice and rats. Furthermore, urinary NGAL was evident even after very mild "subclinical" renal ischemia, despite normal serum creatinine levels. Urinary NGAL detection also far preceeded the appearance of traditional markers in the urine, including β2-microglobulin and NAG.

The upregulation and urinary excretion of NGAL may represent a rapid response of renal tubule cells to a variety of insults, and the detection of NGAL in the urine may represent a widely applicable noninvasive clinical tool for the early diagnosis of tubule cell injury.

NGAL is a sensitive, noninvasive urinary biomarker for renal tubular cell injuries, including renal ischemia and nephrotoxemia. The examination of the expression of NGAL in the urine of patients with acute, mild and early forms of renal tubular cell injury, using the rapid and simple detection methods and kits of the present disclosure, can alert and enable clinicians to institute timely interventional efforts in patients experiencing acute renal failure, and to alert clinicians to institute maneuvers aimed at preventing progression in patients with subtle, subclinical renal tubular cell injuries (such as a nephrotoxins, kidney transplants, vascular surgery, and cardiovascular events) to overt ARF.

In the United States alone, there are approximately 16,000 kidney transplants performed every year. This number has been steadily increasing every year. About 10,000 of these are cadaveric kidney transplants, and are at risk for ARF. Each of these patients would benefit enormously from serial NGAL measurements, which could represent routine care.

Ischemic renal injury has also been associated with open heart surgery, due to the brief interruption in blood flow that is inherent in this procedure. The number of open heart surgeries performed annually can be estimated. In any moderately busy adult hospital, approximately 500 such operations are performed every year. Given that there are at least 400 such moderately busy hospitals in the United States alone, one can conservatively estimate that 200,000 open heart surgeries are performed every year. Again, serial NGAL measurements would be invaluable in these patients, and would represent the standard of care.

### EXPERIMENTAL PROCEDURES

### 1. Mouse models of renal ischemia-reperfusion injury:

We utilized well-established murine models of renal ischemia-reperfusion injury, in which the structural and functional consequences of brief periods of renal ischemia have been previously documented (3-7). Briefly, male Swiss- Webster mice (Taconic Farms, Germantown, NY) weighing 25-35 g were housed with 12:12 hour lightdark cycle and were allowed free access to food and water. The animals were anesthetized with sodium pentobarbital (50 mg/kg intraperitoneally), and placed on a warming table to maintain a rectal temperature of 37°C. Three separate protocols were employed: (a) unilateral ischemia, (b) bilateral ischemic with ARF, and (c) bilateral mild subclinical ischemia. For the first set of (unilateral ischemia) experiments, the left renal pedicle was occluded with a non-traumatic vascular clamp for 45 min, during which time the kidney was kept warm and moist. The clamp was then removed, the kidney observed for return of blood flow, and the incision sutured. The mice were allowed to recover in a warmed cage. After 0, 3, 12, or 24 hours of reperfusion, the animal was re-anesthetized, the abdominal cavity was opened, and blood obtained via puncture of the inferior vena cava for measurement of serum creatinine by quantitative colorimetric assay kit (Sigma, St. Louis, MO). The mice were killed with intraperitoneal pentobarbital. The left ventricle was then perfused with 10 ml of IX PBS, and then with 10 ml of 4% paraformaldehyde in PBS to achieve in situ fixation of the kidneys. Both kidneys were harvested (the right kidney served as a control for each animal). At least three separate animals were examined at each of the reflow periods. One half of each kidney was snap frozen in liquid nitrogen and stored at -70°C until further processing; a sample was fixed in formalin, paraffin-embedded, and sectioned (4 µm). Paraffin sections were stained with hematoxylin-eosin and examined histologically. The clamped kidneys displayed the characteristic morphologic changes resulting from ischemia-reperfusion injury, as previously published by others (3-6) and us (2). The other half of each kidney was embedded in OCT compound (Tissue-Tek) and frozen sections (4 µm) obtained for immunohistochemistry.

For the second set of (bilateral ischemia) experiments, both kidneys were clamped for 30 min, and examined as various reflow periods as detailed above. This group of eight animals was designed to represent ARF, and displayed a significant elevation in serum creatinine at 24 hours following the injury.

For the third set of (bilateral mild subclinical ischemia) experiments, both kidneys of separate animals were clamped for 5, 10, or 20 min only, and examined at various reperfusion periods as above. This very mild degree of injury was designed to simulate subclinical renal ischemia, and mice in this group did not display any elevations in serum creatinine measured at 24 hours following the injury. 2. Rat model of renal ischemia-reperfusion injury:

We utilized well-established rodent models of renal ischemia-reperfusion injury (2). Briefly, male Sprague-Dawley rats weighing 200-250 g (Taconic Farms, Germantown, NY) were anesthetized with ketamine (150 µg/g) and xylazine (3 µg/g), and subjected to bilateral renal artery occlusion with microvascular clamps for 30 min as detailed in the mouse protocol. Timed urine collections were obtained at 3, 6, 9, 12 and 24 h of reperfusion, and blood was collected for creatinine measurement at the time of killing (24 h).

### 3. RNA isolation:

Mouse whole kidney tissues (or cultured human proximal tubule cells, see below) were disrupted with a Tissue Tearor (Biospec Products, Racine, WI). Total RNA from control and ischemic kidneys was isolated using the RNeasy Mini Kit (Qiagen, Valencia, CA), and quantitated by spectrophotometry.

### 4. Microarray Procedures:

Detailed descriptions of microarray hardware and procedures have been previously published (3). Briefly, for each experiment, 100 µg of purified total mouse kidney RNA was reverse transcribed with Superscript II reverse transcriptase (Life Technologies, Rockville, MD) in the presence of Cy3-dUTP (Amersham, Piscataway, NJ) for controls and Cy5-dUTP for ischemic samples. The cDNA samples were purified using a Microcon YM-50 filter (Millipore, Madison, WI), and hybridized to microarray slides containing 8,979 unique sequence-verified mouse probes (3). Three separate animals were examined for each of the reflow periods, and at least two independent microarray experiments were performed for each of the animals. The array slides were scanned using a microarray scanner (GenePix 4000B, Axon Instruments, Foster City, CA) to obtain separate TIFF images for Cy3 and Cy5 fluorescence. The signal intensities for Cy3 and Cy5 were determined for individual genes using the GenePix Pro 3.0 data extraction software (Axon Instruments). Quality control and data analysis was completed as previously described (3).

### 5. Semi-quantitative Reverse Transcription-Polymerase Chain Reaction (RT-PCR):

An equal amount (1 µg) of total RNA from control and experimental mouse kidneys was reverse transcribed with Superscript II reverse transcriptase (Life Technologies) in the presence of random hexamers according to the manufacturer's instructions. PCR was accomplished using a kit (Roche, Indianapolis, IN) and the following primers:
Mouse NGAL sense 5'-CACCACGGACTACAACCAGTTCGC-3' ;
Mouse NGAL antisense 5'-TCAGTTGTCAATGCATTGGTCGGTG-3' ;
Human NGAL sense 5'-TCAGCCGTCGATACACTGGTC-3' ; and
Human NGAL antisense 5'-CCTCGTCCGAGTGGTGAGCAC-3'.

Primer pairs for mouse and human β-actin and glyceraldehyde-3-phosphate dehydrogenase (GAPDH) were obtained from Clontech (La Jolla, CA). Mock reactions devoid of cDNA served as negative controls. PCR products were analyzed by agarose gel electrophoresis followed by staining with ethidium bromide, and quantitated by densitometry. Fold changes in NGAL mRNA expression in ischemic versus control kidneys were expressed following normalization for β-actin or GAPDH amplification.

### 6. Immunohistochemistry:

Frozen sections were permeabilized with 0.2% Triton X-100 in PBS for 10 min, blocked with goat serum for 1 hr, and incubated with primary antibody to NGAL (1 :500 dilution) for 1 hr. Slides were then exposed for 30 min in the dark to secondary antibodies conjugated with Cy5 (Amersham, Arlington Heights, IL), and visualized with a fluorescent microscope (Zeiss Axiophot) equipped with rhodamine filters.

For co-localization of NGAL with Rabl l, serial sections were first incubated with NGAL antibody or a monoclonal antibody to Rabl 1 (1 :500 dilution; Transduction Laboratories), then with secondary antibodies conjugated with either Cy5 (for NGAL) or Cy3 (for Rabl l) and visualized with rhodamine or fluorescein filters, respectively. For co-localization of NGAL with proliferating cell nuclear antigen (PCNA), sections were co-incubated with NGAL antibody and a monoclonal antibody to PCNA (1:500 dilution; Upstate), and was detection accomplished by immunoperoxidase staining (ImmunoCruz Staining System, Santa Cruz Biotechnology). For the TUNEL assay, we used the ApoAlert DNA Fragmentation \Assay Kit Clontech). Paraffin sections were deparaffmized through xylene and descending grades of ethanol, fixed with 4% formaldehyde/PBS for 30 min at 4°C, permeabilized with proteinase K at room temperature for 15 min and 0.2% triton X-100/PBS for 15 min at 4°C, and incubated with a mixture of nucleotides and TdT enzyme for 60 min at 37°C. The reaction was terminated with 2X SSC, and the sections washed with PBS and mounted with Crystal/mount (Biomeda, Foster City, CA). TUNEL-positive apoptotic nuclei were detected by visualization with a fluorescence microscope.

### 7. Urine Collection:

Mice or rats were placed in metabolic cages (Nalgene, Rochester, NY), and urine collected before and every hour after bilateral renal artery occlusion. Urine samples were centrifuged at 5000 x g to remove debris, and the supernatant analyzed by Western blotting. Urinary creatinine was measured by quantitative colorimetric assay kit (Sigma) to normalize samples for urinary NGAL determination. A colorimetric assay kit for the determination of N-acetyl-β-D-glucosaminidase (NAG) in the urine was obtained from Roche.

### 8. Cell Culture:

Human renal proximal tubular epithelial cells (RPTEC) were obtained from Clonetics (San Diego, CA). Cells were grown in Renal Epithelial Cell Basal Medium supplemented with REGM complex (0.5 µl/ml hydrocortisone, 10 pg/ml hEGF, 0.5 µg/ml epinephrine, 6.5 pg/ml triiodothyronine, 10 µg/ml transferrin, 5 µg/ml insulin, 1 µg/ml gentamicin sulfate, and 2% FBS), as recommended by the manufacturer.

### 9. Mild ATP depletion of cultured cells:

We modified previously described protocols of in vitro ischemia by ATP depletion with inhibitors of oxidative phosphorylation (8, 9). On the second day post-confluence, RPTEC cells were incubated with 1 µm antimycin A (Sigma) for varying periods of time up to 6 h. We have previously shown that this results in mild partial reversible ATP depletion, and no loss of cell viability, in other types of cultured renal epithelial cells such as MDCK (8) and 786-0 (9) cells. ATP levels were monitored using a luciferase-based assay kit (Sigma), and expressed as a percentage of control values. Cells were harvested at various time points of ATP depletion, and analyzed for NGAL mRNA expression by RT-PCR and NGAL protein expression by Western analysis. The secretion of NGAL into the culture medium was also monitored.

### 10. Mouse Model of Cisplatin Nephrotoxicity

We utilized a well-established murine model in which the structural and functional consequences of cisplatin-induced nephrotoxicity have been previously documented (12-14, 18). Briefly, male Swiss- Webster mice (Taconic Farms, Germantown, NY) weighing 25-30 g were housed with 12:12 hour light: dark cycle and were allowed free access to food and water. Mice were given a single intraperitoneal injection of cisplatin, in the dose of either 5 µg/kg or 20 µg/kg body weight. It has been previously shown that the larger dose results in tubule cell necrosis and apoptosis, and impaired renal function within 3-4 days after the cisplatin injection (12-14, 18). Animals were placed in metabolic cages (Nalgene, Rochester, NY), and urine collected before and at various time points (3, 12, 24, 48, 72 and 96 h) following cisplatin. At similar time points, the animals were anesthetized with sodium pentobarbital (50 mg/kg intraperitoneally), the abdominal cavity opened, and blood obtained via puncture of the inferior vena cava for measurement of serum creatinine using a quantitative colorimetric assay kit (Sigma, St. Louis, MO). The mice were sacrificed, the kidneys perfusion fixed in situ with 4% paraformaldehyde in PBS, and both kidneys harvested. One half of each kidney was snap frozen in liquid nitrogen and stored at -70° C until further processing; a sample was fixed in formalin, paraffin-embedded, and sectioned (4 mm). Paraffin sections were stained with hematoxylin-eosin and subjected to the TUNEL assay. The rest was processed for Western blotting. Whole kidneys were homogenized in ice-cold lysis buffer (20 mM Tris, pH 7.4, 250 mM sucrose, 150 mM NaCl, 1% NP-40, and IX Complete® protease inhibitors) using a Polytron homogenizer. The homogenates were incubated on ice for 30 min, centrifuged at 1,000 x g for 5 min at 4° C to remove nuclei and cellular debris, and analyzed for protein content by the Bradford assay (Bio-Rad, Hercules, CA). The other half of each kidney was embedded in OCT compound (Tissue-Tek) and frozen sections (4 µm) obtained for immunohistochemistry.

### 11. Expression, purification, and Western Bloltting of recombinant murine NGAL

Full length mouse NGAL cDNA was cloned into the pGEX expression vector (Pharmacia, Nutley, NJ), expressed as a fusion protein with glutathione-S-transferase (GST) in bacteria, and purified using glutathione-sepharose columns (Amersham) followed by fhrombin cleavage as previously described (16, 19, 20). Proteins were analyzed by SDS-PAGE followed by Coomassie blue staining or by Western blotting with a polyclonal antibody to NGAL. Protein concentrations were determined using the Bradford assay (Bio-Rad, Hercules, CA).

### 12. Quantitation of urinary NGAL by Western Blotting

The amount of NGAL in the urine was determined by comparison with defined standards of recombinant purified NGAL. Densitometric analysis of Western blots using known concentrations of recombinant NGAL and known volumes of urine were performed under identical conditions of transfer and exposure.

All chemicals were purchased from Sigma unless otherwise specified. For Western blotting, protein concentrations were determined by the Bradford assay (Bio-Rad, Hercules, CA), and equal amounts of total protein were loaded in each lane. Monoclonal antibody to α-tubulin (Sigma) was used at 1 :10,000 dilution for confirmation of equal protein loading, and polyclonal antibody to NGAL was used at 1 :500 (15), unless otherwise specified. Immunodetection of transferred proteins was achieved using enhanced chemiluminescence (Amersham), unless otherwise specified.

### EXAMPLE 1

NGAL is a small protease-resistant, secreted polypeptide that is detectable in the urine. The marked upregulation of NGAL mRNA and protein levels has been shown in the early post-ischemic mouse kidney. NGAL protein expression was detected predominantly in proximal tubule cells, in a punctate cytoplasmic distribution reminiscent of a secreted protein. Indeed, NGAL was easily and rapidly detected in the urine (in the very first urine output) following ischemic injury in both mouse and rat models of ARF, at which time no leukocytic infiltration of the kidney was observed. The origin of NGAL from tubule cells was further confirmed in cultured human proximal tubule cells subjected to in vitro ischemic injury, where NGAL mRNA was markedly and promptly induced in the cells, and NGAL protein readily detectable in the culture medium within one hour of mild ATP depletion. Our results indicate that NGAL may represent a novel early urinary biomarker for ischemic renal injury.

### Identification of novel genes upregulated early after renal ischemia-reperfusion injury:

A genome-wide search for transcripts induced soon after renal ischemia-reperfusion injury in a mouse model identified seven early biomarkers. Three separate mice were examined at each of the reperfusion periods (3, 12, and 24 h), and at least two separate microarray experiments were performed for each animal examined. A comparison of the transcriptome profiles of control and ischemic kidneys yielded a small subset of seven genes that were consistently induced greater than 10-fold. One of these transcripts, cysteine rich protein 61 (Cyr61), has very recently been confirmed to be induced by renal ischemia (1). Surprisingly, the behavior of the other six differentially expressed genes is novel to the ARF literature. We chose to further characterize one of these previously unrecognized genes, namely neutrophil gelatinase-associated lipocalin (NGAL).

### Characterization of the Animal Models of Early Renal Failure:

Ischemia-reperfusion injury murine models were used in which the structural and functional consequences of brief periods of renal ischemia have been documented (3-7). The characteristic histopathologic features of ischemic injury were readily evident in the 24-h reperfusion samples after both unilateral (45 min) and bilateral (30 min) ischemia. These included a loss of brush border membranes, tubular dilation, flattened tubular epithelium, luminal debris, and an interstitial infiltrate (Figure 1). The presence of apoptotic cells was documented using the TUNEL assay. Apoptosis was predominantly localized to distal tubular cells and ascending limb of Henle's loop, both in detached cells within the lumen as well as attached cells. Occasional proximal tubular cells were also apoptotic, but the glomeruli were essentially devoid of apoptosis. No TUNEL-positive cells were detected in the control kidneys or in the ischemic samples where TdT was omitted (not shown). The above histologic and apoptotic changes were absent from kidneys subjected to milder degrees of ischemia (5, 10, or 20 min of bilateral ischemia; not shown). The serum

creatinine levels were reflective of the histopathologic changes observed. Thus, mice with unilateral renal ischemia or mild degrees of subclinical bilateral ischemia displayed serum creatinine levels that were indistinguishable from control animals, whereas mice with bilateral ischemia for 30 min showed a significant elevation of serum creatinine (Figure 1).

### NGAL mRNA is markedly induced in the early post-ischemic kidney:

By microarray analysis, NGAL was found to be consistently induced 3.2 ± 0.5 fold, 11.1 ± 1.2 fold, and 4.3 ± 0.6 fold at 3, 12, and 24 h of reperfusion in the ischemic mouse kidney when compared to the control kidneys from the same animal (mean +/- SD from three animals at each time point). This finding was confirmed by semi-quantitative RT-PCR, using a normalization protocol with both β-actin and GAPDH. No significant changes in mRNA expression of either β-actin or GAPDH were noted at any of the reperfusion periods examined, as previously described (3). However, using mouse-specific primers, we detected a significant upregulation of NGAL mRNA expression (4.1 ± 0.5 fold, 9 ± 0.6 fold, and 4.2 ± 0.4 fold at 3, 12, and 24 h of reperfusion respectively, where values represent mean +/- SD from three separate animals). These results are illustrated in Figure 1, and are in overall agreement with the changes detected by transcriptome analysis.

### NGAL protein is markedly over-expressed in the proximal tubules of early ischemic mouse kidneys:

The post-ischemic expression of NGAL protein in the kidney parallels that of the mRNA. By Western analysis, NGAL was just detectable as a 25 kDa immunoreactive peptide in control mouse kidneys. The identity of this band as NGAL was established in a separate set of experiments, where pre-incubation of the primary antibody with recombinant mouse lipocalin completely blocked this immunoreactivity (not shown). In the unilateral ischemic experiments, NGAL expression was induced 3-4 fold by densitometry in the ischemic kidney from three separate animals within 3 h of injury, as shown in Figure 2, Panel A. This response was dramatically enhanced in the bilateral ischemia experiments from eight separate animals. NGAL in these mice was induced threefold after 3 h of reperfusion, peaked at greater than 12-fold in the 24-h samples, and declined to normal levels by the 72-h recovery period (Figure 2, Panel B).

Using immunohistochemical techniques, NGAL protein was barely detectable in control mouse kidneys, but is upregulated predominantly in proximal tubules within 3 h of ischemia as illustrated in Figure 3. Identification of proximal tubules in these sections was based on the presence of a brush border membrane, ratio of nuclear to cell size, and cellular morphology. The induced NGAL appeared in a punctate cytoplasmic distribution within proximal tubule cells, reminiscent of a secreted protein. This pattern of expression was identical in both unilateral and bilateral models of ischemia-reperfusion injury, and was consistently evident in every animal studied. The glomeruli were devoid of NGAL expression, and no NGAL-expressing neutrophils were evident. Because NGAL has been shown in cultured Wilms tumor kidney cells to co-localize at least in part with endosomes (11), the distribution of NGAL and Rabl 1 (a marker of late recycling endosomes) was examined in serial kidney sections. Merged images showed a significant co-localization of NGAL with Rabl 1 (not shown). To determine the functional significance of enhanced NGAL expression after ischemia, serial kidney sections were examined for NGAL expression, TUNEL-positive nuclei, or PCNA-positive nuclei. Whereas tubule cells overexpressing NGAL were not TUNEL-positive (not shown), a significant co-localization of NGAL and PCNA was evident in the proliferating and regenerating cells at the 48-h reflow period (not shown).

### NGAL protein is easily detected in the urine immediately after induction of ARF in mice:

This experiment demonstrates the utility of detecting urinary NGAL as an early noninvasive biomarker of ischemic renal injury. Using urinary creatinine concentrations to equalize for sample loading, NGAL was absent from the urine prior to ischemia. In striking contrast, NGAL was manifest as a 25 kDa band within 2 h of the injury (in the very first urine output following ischemia) in all animals examined, as shown in Figures 4A and 4B. The identity of this band as NGAL was established in a separate set of experiments, where pre-incubation of the primary antibody with recombinant mouse lipocalin completely blocked this immunoreactivity (not shown). NGAL was easily detectable in as little as 1 µl of unprocessed urine by Western analysis, and persisted for the entire duration examined (24 h of reperfusion). We then compared urinary NGAL excretion with that of previously established markers of injury, such as β2-microglobulin and NAG. Whereas urinary NGAL was evident within 2 h of ischemia, β2-microglobulin was detectable in the same urinary samples only after 12 h of unilateral (Figure 4, Panel A) and 8 h of bilateral ischemia (Figure 4, Panel B). Similarly, urinary NAG excretion was significantly increased only after 12 h of unilateral (bottom panel of Figure 4, Panel A) and 8 h of bilateral ischemia (bottom panel of Figure A, panel B) when compared with nonischemic control animals.

### NGAL protein is easily detected in the urine even after mild renal ischemia in mice:

In order to determine the sensitivity of urinary NGAL detection in the absence of overt ARF, we employed protocols whereby separate sets of mice were subjected to only 5, 10, or 20 min of bilateral renal artery occlusion. These studies were designed to assess urinary NGAL excretion following mild subclinical renal ischemia. Serum creatinine measured after 24 h of reflow was within normal limits in all these mice. Strikingly, NGAL was easily detected in as little as 1 µl of unprocessed urine in these animals (Figure 5), although its appearance was somewhat delayed compared to animals with ARF. Thus, while 30 min of bilateral ischemia resulted in urinary NGAL excretion within 2 h (Figure 4), mice with 20 or 10 min of bilateral ischemia manifested urinary NGAL after 4 h, and those with 5 min of ischemia excreted NGAL only after 6 h (Figure 5). Thus, the appearance NGAL in the urine appears to be related to the dose and duration of renal ischemia.

### EXAMPLE 2

### NGAL protein is easily detected in the urine immediately after induction of ARF in rats:

Since a debate exists regarding species differences in the responses to renal artery occlusion (10), we next examined the behavior of NGAL in a different animal model, namely a well-established rat model of renal ischemia-reperfusion injury. Using urinary creatinine concentrations to equalize for sample loading, NGAL was absent from the urine prior to rat renal ischemia. In marked contrast, NGAL was manifest as a 25 kDa immunoreactive peptide within 3 h of the injury (in the very first urine output following ischemia), as shown in Figure 6. In comparison, the serum creatinine in this model of ischemic injury was elevated only after 24 h of reperfusion (not shown). Once again, NGAL was detectable in 1 µl of unprocessed urine and persisted for the entire duration examined (24 h of reperfusion).

### EXAMPLE 3

### NGAL mRNA is induced in cultured human proximal tubule cells after early mild ischemia:

In order to confirm the origin of NGAL from ischemic proximal tubule cells, we modified previously described protocols of in vitro ischemia by ATP depletion in cultured human proximal tubule cells (RPTEC). Incubation in 1 µm antimycin resulted in a mild partial ATP depletion to about 83 ± 3% of control within 1 h, with a more gradual decrease to about 75 ± 3% of control by 6 h (mean +/- SD from four experiments). No morphological consequences of this mild ATP depletion were discernible. NGAL mRNA was just detectable in resting cells. However, following partial ATP depletion, a rapid and duration-dependent induction of NGAL mRNA was evident by RT-PCR, as shown in Figure 7.

### NGAL protein is easily detected in the medium after early ischemia in vitro:

We next examined NGAL protein expression in RPTEC cells and the culture medium following mild ATP depletion. NGAL protein was detectable in control RPTEC cells, and its expression increased after ATP depletion in a duration-dependent manner, as shown in Figure 7. No NGAL immunoreactive protein was found in the culture medium from control cells, but NGAL was easily detectable within 1 hour of mild ATP depletion. Further increases in NGAL protein abundance were noted related to the duration of ATP depletion. These results suggest that the induced NGAL protein is rapidly secreted into the medium, analogous to the swift appearance of NGAL in the urine following renal ischemia in vivo.

### EXAMPLE 4

### NGAL protein is easily detected in the urine early after mild renal nephrotoxemia in mice:

To determine whether nephrotoxemia results in the expression of the NGAL protein in the urine, thereby suggesting its utility as an early noninvasive biomarker of nephrotoxic renal injury, cisplatin-induced nephrotoxemia was induced in mice. In an established mouse model of cisplatin nephrotoxicity, NGAL was easily detected in the urine within 1 d of cisplatin administration (Figure 8A, bottom track). In contrast, urinary β2-microglobulin was barely detectable after 2 d and could not be reliably detected until day 4 to 5 after cisplatin (Figure 8, Panel A, top track). Similarly, increased urinary NAG excretion was not evident until days 4 and 5 after cisplatin administration (Figure 8, Panel B).

### Cisplatin nephrotoxicity is characterized by apoptosis and necrosis in renal tubule cells:

Mice were given a single intraperitoneal injection of cisplatin, in the dose of either 5 mg/kg or 20 mg/kg body weight. Results in control mice and those receiving the larger dose of cisplatin are shown in Figure 9. The larger dose resulted in tubule cell necrosis, as evidenced by the presence of tubular dilatation, luminal debris, and flattened epithelium in sections stained with hematoxylin-eosin (upper center panel). Also documented were tubule cells undergoing programmed cell death, indicated by condensed intensely-stained nuclei (upper right panel). This was confirmed by TUNEL assay, which showed the condensed, fragmented nuclei characteristic of apoptosis (lower center and right panels). No necrosis or apoptosis was detected in the control kidneys (upper and lower left panels). Kidneys from mice treated with the smaller dose of cisplatin also appeared normal, similar to untreated controls (not shown). Figure 9 is representative of 5 separate experiments.

### NGAL protein is rapidly induced in kidney tubules by cisplatin:

Since NGAL is induced following ischemic injury to the kidney (17), the response to cisplatin-induced nephrotoxic damage was determined. By Western analysis, NGAL was barely detectable in kidney lysates from control mice, but was rapidly induced within 3 hours of cisplatin administration (20 mg/kg), as illustrated in Figure 10. There was a duration-dependent increase in kidney NGAL expression, with a peak at 48 hours and a persistent upregulation for up to 96 hours. These results were confirmed by immunofluorescence staining, shown in Figure 11. Kidneys harvested at 3 (3h) (top right panel) and 12 (12h) (bottom left panel) hours after cisplatin injection showed induction of NGAL protein. Also shown in Figure 11 is a high power magnification image of the section harvested at 12 hours (HP) (bottom right panel). The arrow on the bottom left panel indicates the region shown in the HP image. NGAL was induced within 3 hours of cisplatin injection, predominantly in proximal tubule cells, but was absent in cells from control mice (Con) (top left panel). Identification of proximal tubules in these sections was based on the presence of a brush border membrane, ratio of nuclear to cell size, and cellular morphology. The induced NGAL appeared in a punctate cytoplasmic distribution within proximal tubule cells, reminiscent of a secreted protein. The induced NGAL appeared in a punctate cytoplasmic distribution within proximal tubule cells, reminiscent of a secreted protein. This pattern of expression was similar to that observed in mouse models of ischemia-reperfusion injury (17). The glomeruli were devoid of NGAL expression, and no NGAL-expressing neutrophils were evident. Figure 11 represents 5 animals at each time point.

### NGAL protein is easily detected in the urine after high-dose cisplatin:

NGAL protein was detected in the urine following high dose cisplatin (20 mg/kg), thereby demonstrating its utility as an early noninvasive biomarker of nephrotoxic renal injury. Using urinary creatinine concentrations to equalize for sample loading, NGAL was essentially absent from the urine prior to ischemia. In striking contrast, urinary NGAL was easily detected within 3 hours of cisplatin injury (20 µg/kg) in all animals examined, as shown in Figure 12 (top panel). The identity of this band as NGAL was established in a separate set of experiments, in which pre-incubation of the primaiy antibody with recombinant mouse lipocalin completely blocked this immunoreactivity (not shown). NGAL was easily detectable in as little as 5 µl of unprocessed urine by Western analysis. There was a duration-dependent increase in urinary NGAL excretion, with a peak at 48 hours and a persistent upregulation for up to 96 hours. We then compared urinary NGAL excretion with that of previously established markers of injury such as NAG. Whereas urinary NGAL was evident within 3 hours of cisplatin, urinary NAG excretion was significantly increased only after 96 hours of injury (center panel). Furthermore, assessment of renal function by serum creatinine measurements showed a significant change only after 96 hours of cisplatin (bottom panel). The figure represents five independent experiments at each time point.

### NGAL protein is detected in the urine even after low dose cisplatin:

Separate sets of mice were subjected to only 5 µg/kg of cisplatin injections in order to determine the sensitivity of urinary NGAL detection following sub-clinical nephrotoxic injury, shown in Figure 13. NGAL was detectable in as little as 5 µl of unprocessed urine in these animals (top panel), although its appearance appeared to be quantitatively less compared to animals with 20 µg/kg cisplatin (Figure 12, top panel). Thus, the appearance NGAL in the urine correlates with the dose of nephrotoxin. Whereas urinary NGAL excretion was evident within 3 hours of cisplatin, urinary NAG excretion in this group of animals was not significantly increased even after 96 hours of injury (center panel). Furthermore, assessment of renal function by serum creatinine measurements showed that serum creatinine was not significantly altered even after 96 hours of low-dose cisplatin (bottom panel). This example demonstrates that NGAL is a more sensitive marker of renal nephrotoxcicity than ones currently in use.

### Urinary NGAL excretion following cisplatin is dose- and duration-dependent:

Urinary NGAL excretion was quantitated to determine its utility as an indicator of the severity of a renal injury following cisplatin administration, shown in Figure 14. This required the expression and purification of known quantities of NGAL for use as a standard. Analysis of recombinant NGAL protein by SDS-PAGE followed by Coomassie blue staining showed a single protein band of the appropriate size (top left panel). Western blotting of aliquots of known concentration revealed a linear increase in signal intensity at the 3-100 ng/ml range (top right panel). The amount of NGAL in the urine was then determined by comparison with these defined standards of recombinant purified NGAL. Following 20 µg/kg cisplatin, there was a duration-dependent increase in urinary NGAL excretion (bottom panel). A similar, although somewhat blunted, response was evident in animals treated with cisplatin doses resulting in sub-clinical nephrotoxic injury.

### EXAMPLE 5

Urine samples were obtained from patients two hours after kidney transplantation, which is a predictable human model of ischemic renal injury, shown in Figure 15. Patients (n=4) receiving cadaveric kidneys that are stored on ice for a period of time prior to implantation, had increased urinary NGAL that was easily quantified by Western blot and ELISA, compared to patients (n=6) receiving kidneys from living related donors (panel A). There was a significant correlation between urinary NGAL and cold ischemia time, indicating that NGAL excretion is proportional to the degree of renal injury (panel B) (r =0.98, Spearman analysis). There was also a significant correlation between urinary NGAL and the peak serum creatinine (panel C). (r =0.96, Spearman analysis). It is important to emphasize that urinary NGAL measured within two hours of transplantation was predictive of ARF as reflected by serum creatinine peak, which occurred several days later. Urine from normal human controls or from patients with chronic renal failure contained almost undetectable amounts of NGAL, indicating that upregulation of urinary NGAL is specific to acute renal injury (not shown). Also, urine from patients with urinary tract infections and kidney transplant rejection (two neutrophil-related disorders) contained only minimal quantities of NGAL (not shown), easily distinguishable from the significantly greater quantities in cadaveric kidney transplants (>100 ng/ml). These data demonstrate that NGAL is a novel early urinary biomarker for acute renal injury following kidney transplantation.

### EXAMPLE 6

Serial urine samples were obtained prospectively from fifteen patients after open heart surgery, with results shown in Figure 16. Urinary NGAL was quantified by Western blot and ELISA and found to be elevated in five of these fifteen patients (panel A). Each line represents one patient. The % change in serum creatinine from baseline is shown on the right of panel A. The same five patients developed post-operative acute renal failure, defined as a 50% or greater increase in serum creatinine from baseline, yielding an incidence rate of about 33%. In the 10 patients who did not develop acute renal failure, there was small early increase in urinary NGAL excretion (2 hour values of 6.0±2.0 ng/mg creatinine) that rapidly normalized to almost undetectable levels within 12 hours post surgery (panel A). In marked contrast, patients who subsequently developed acute renal failure displayed a greater than 10-fold increase in the 2 hour value for urinary NGAL (75+10 ng/mg creatinine), and a greater than 20-fold increase in the 4 hour value for urinary NGAL (120+12 ng/mg creatinine). There was a correlation between the quantity of urinaiy NGAL and cardiopulmonary bypass time, indicating that NGAL excretion is proportional to the degree of renal injury (panel B). (r=0.76, Spearman analysis) There was also a significant correlation between urinary NGAL and the peak serum creatinine (panel C). (r=0.66, Spearman analysis) It is important to once again emphasize that urinary NGAL measured within two hours of cardiac surgery was predictive of ARF as reflected by serum creatinine peak, which occurred several hours or even days later. These data show that NGAL is a novel early urinary biomarker for acute renal injury following open heart surgery, and its quantitation is predictive of acute renal failure in this highly susceptible population.

### REFERENCES

1. Muramatsu Y, Tsujie M, Kohda Y, Pham B, Perantoni AO, Zhao H, Jo S-K, Yuen PST, Craig L, Hu X, Star RA: Early detection of cysteine rich protein 61 (CYR61, CCN1) in urine following renal ischemia reperfusion injury. Kidney Int 62:1601-1610, 2002
2. Yoshida T, Kurelia M, Beato F, Min H, Ingelfinger JR, Stears RL, Swinford RD, Gullans SR, Tang S-S: Monitoring changes in gene expression in renal ischemia-reperfusion in the rat. Kidney Int 61:1646-1654, 2002
3. Supavekin S, Zhanh W, Kucherlapati R, Kaskel FJ, Moore LC, Devarajan P: Differential gene expression following early renal ischemia-reperfusion. Kidney Int 63:1714-1724, 2003.
4. Nogae S, Miyazaki M, Kobayashi N, Saito T, Abe K, Saito H, Nakane PK, Nakanishi Y, Koji T: Induction of apoptosis in ischemia-reperfusion model of mouse kidney: Possible involvement of Fas. J Am Soc Nephrol 9:620-631, 1998
5. Daemen MARC, Van de Ven MWCM, Heineman E, Buurman WA: Involvement of endogenous interleukin-10 and tumor necrosis factor- D in renal ischemia-reperfusion injury. Transplantation 67:792-800, 1999
6. Kelly KJ, Plotkin Z, Dagher PC: Guanosine supplementation reduces apoptosis and protects renal function in the setting of ischemic injury. J Clin Invest 108:1291-1298, 2001
7. Burne NJ, Rabb H: Pathophysiological contributions of fucosyltransferases in renal ischemia reperfusion injury. J Immunol 169:2648-2652, 2002
8. Feldenberg LR, Thevananther S, del Rio M, De Leon M, Devarajan P: Partial ATP depletion induces Fas- and caspase-mediated apoptosis in MDCK cells. Am J Physiol 276 (Renal physiol 45):F837-F846, 1999
9. Devarajan P, De Leon M, Talasazan F, Schoenfeld AR, Davidowitz EJ, Burk RD: The von Hippel-Lindau gene product inhibits renal clell apoptosis via Bcl-2-dependent pathways. J Biol Chem 276:40599-40605, 2001
10. Molitoris BA, Weinberg JM, Venkatachalam MA, Zager RA, Nath KA, Goligorsky MS: Acute renal failure II. Experimental models of acute renal failure: imperfect but indispensable. Am J Physiol Renal Physiol 278:F1-F12, 2000
11. Kjeldsen L, Cowland JB, Borregaard N: Human neutrophil gelatinase-associated lipocalin and homologous proteins in rat and mouse. Biochim Biophys Acta 1482:272-283, 2000
12. Megyesi J, Safirstein RL, Price PM: Induction of p21WAFl/CIP/SDl in kidney tubule cells affects the course of cisplatin-induced acute renal failure. J Clin Invest 101: 777-782, 1998
13. Shiraishi F, Curtis LM, Truong L, Poss K, Visner GA, Madsen K, Nick HS, Agarwal A: Heme oxygenase-1 gene ablation or expression modulates cisplatin-induced renal tubular apoptosis. Am J Physiol 278: F726-F736, 2000
14. Ramesh G, Reeves WB: TNF-α mediates chemokine and cytokine expression and renal injury in cisplatin nephrotoxicity. J Clin Invest 110: 835-842, 2002
15. Muramatsu Y, Tsujie M, Kohda Y, Pham B, Perantoni AO, Zhao H, Jo S-K, Yuen PST, Craig L, Hu X, Star RA: Early detection of cysteine rich protein 61 (CYR61, CCN1) in urine following renal ischemic reperfusion injury. Kidney Int 62: 1601-1610, 2002
16. Yang J, Goetz D, Li J-Y, Wand W, Mori K, Setlik D, Du T, Erdjument-Bromage H, Tempst P, Strong R, Barasch J: An iron delivery pathway mediated by a lipocalin. Mol Cell 10:1045-1056, 2002
17. Mishra J, Ma Q, Prada A, Mitsnefes M, Zahedi K, Yang J, Barasch J, Devarajan P: Identification of NGAL as a novel urinary biomarker for ischemic injury. J Am Soc Nephrol 14:2534-2543, 2003
18. Tsuruya K, Ninomiya T, Tokumoto M, Hirakawa M, Masutani K, Taniguchi M, Fukuda K, Kanai H, Kishihara K, Hirakata H, lida M: Direct involvement of the receptor-mediated apoptotic pathways in cisplatin-induced renal tubularcell death. Kidney Int 63: 72-82, 2003
19. Bundgaard J, Sengelov H. Borregaard N, Kjeldsen L: Molecular cloning and expression of a cDNA encoding Ngal: a lipocalin expressed in human neutrophils. Biochem Biophys Res Commun 202: 1468-1475, 1994
20. Del Rio M, Imam A, De Leon M, Gomez G, Mishra J, Ma Q, Parikh S, Devarajan P: The death domain of kidney ankyrin interacts with Fas and promotes Fas-mediated cell death in renal epithelia. J Am Soc Nephrol 15:41-51, 2004

## Claims

1. A method for the detection of a renal tubular cell injury in a human patient caused by an event, the said event being chosen from the group consisting of diminished blood supply to the kidneys, impaired heart function, surgical procedures, patients in intensive care units, and the administration of pharmaceuticals, radiocontrast dyes, or other medicament substances to a human patient, said method comprising the steps of:
1) contacting a urine sample obtained from a human patient with an antibody for a renal tubular cell injury biomarker, the biomarker consisting of NGAL, to allow formation of a complex of the antibody and NGAL; and
2) detecting the antibody-NGAL complex.

2. The method according to Claim 1 wherein a plurality of urine samples from the subject is obtained intermittently.

3. The method according to Claim 2 wherein the urine samples are obtained continuously.

4. The method according to Claim 1 wherein the step of detecting the antibody-NGAL complex comprises contacting the complex with a second antibody for detecting NGAL.

5. The method according to claim 1 for further monitoring the effectiveness of a treatment for a renal tubular cell injury, comprising the steps of:
3) contacting at least one post-treatment urine sample from the human patient experiencing the renal tubular cell injury and receiving treatment therefore, with a capture antibody for NGAL to allow formation of a complex of the antibody and NGAL; and
4) detecting the antibody-NGAL complex in the post-treatment urine sample.

6. The method according to claim 5 wherein the step of detecting the antibody-NGAL complex comprises the steps of:
(4i) separating any unbound material of the urine sample from the capture antibody-NGAL complex;
(4ii) contacting the capture antibody-NGAL complex with a second antibody for detecting NGAL, to allow formation of a complex between NGAL and the second antibody;
(4iii) separating any unbound second antibody from the NGAL-second antibody complex; and
(4iv) detecting the second antibody of the NGAL-second antibody complex.

7. The method according to claim 5, wherein the step 3) comprises the step of contacting the urine sample with a media having affixed thereto the capture antibody.

8. Use of a kit comprising:
1) a means for acquiring a quantity of a urine sample;
2) a media having affixed thereto a capture antibody capable of complexing with NGAL; and
3) an assay for the detection of a complex of NGAL and the capture antibody, in a method according to claim 1.

9. The use according to claim 8, wherein the quantity of the urine sample is less than 1 ml, more typically less than 10 microliters.

10. The use according to claim 8, wherein the acquiring means comprises an implement comprising a surface, the surface comprising the media.

11. The use according to claim 8, wherein the acquiring means comprises a container for accepting the urine sample, wherein the urine-contacting surface of the container comprises the media.

12. The use according to claim 8, wherein the assay comprises an ELISA.

13. The use according to claim 8, wherein the acquiring means comprises an implement comprising a cassette containing the media.

14. The use according to claim 8, wherein the kit is a point-of-care kit.

15. The use according to claim 14, wherein the means for acquiring the urine sample is designed for a quantity of the urine sample of less than 1 ml, more typically less than 10 microliters.

16. The use of a point-of-care kit according to claim 15 wherein the acquiring means comprises an implement comprising a dip-stick, the dip-stick surface comprising the media.

17. The use of the point-of-care kit according to claim 15, wherein the assay comprises a colorimetric dip-stick assay.

18. Use of a competitive enzyme linked immunosorbent assay (ELISA) kit comprising an antibody specific to NGAL to detect its presence in a urine sample of the human being in a method according to claim 1.

19. The use according to claim 18 for assaying a urine sample which can comprise a fluid amount of about 1 milliliter or less.

20. A method of identifying the extent of a renal tubular cell injury, caused by an event, the said event being chosen from the group consisting of diminished blood supply to the kidneys, impaired heart function, surgical procedures, patients in intensive care units, and the administration of pharmaceuticals, radiocontrast dyes, or other medicament substances to a human patient, said method comprising the steps of
1) detecting in at least one urine sample from a human patient the presence of a biomarker for a renal tubular cell injury, said biomarker consisting of NGAL; and
2) determining the extent of the renal tubular cell injury based on the time for onset of the presence of the NGAL in the urine sample, relative to the time of the event.

21. The method according to claim 1, wherein step 1) comprises contacting a urine sample comprising up to 1 milliliter of urine from a human patient with the antibody for NGAL, to allow formation of a complex of the antibody and NGAL.

## Patentansprüche

1. Verfahren zum Nachweis einer Nierentubuluszellenverletzung bei einem menschlichen Patienten, die durch ein Ereignis verursacht wurde, wobei das Ereignis aus der Gruppe ausgewählt ist, die aus verminderter Blutzufuhr zu den Nieren, beeinträchtigter Herzfunktion, operativen Verfahren, Patienten auf Intensivstationen und der Verabreichung von Pharmazeutika, Radiokontrastmitteln oder anderen Arzneimittelsubstanzen an einen menschlichen Patienten besteht, wobei das Verfahren die folgenden Schritte umfasst:
1) Herstellen des Kontakts einer Urinprobe, die von einem menschlichen Patienten gewonnen wurde, mit einem Antikörper für einen Biomarker der Nierentubuluszellenverletzung, wobei der Biomarker aus NGAL besteht, um die Bildung eins Komplexes aus dem Antikörper und NGAL zu ermöglichen; und
2) Nachweisen des Antikörper-NGAL-Komplexes.

2. Verfahren nach Anspruch 1, wobei eine Vielzahl von Urinproben von dem Probanden in Abständen gewonnen wird.

3. Verfahren nach Anspruch 2, wobei die Urinproben fortlaufend gewonnen werden.

4. Verfahren nach Anspruch 1, wobei der Schritt des Nachweisens des Antikörper-NGAL-Komplexes das Herstellen des Kontakts des Komplexes mit einem zweiten Antikörper zum Nachweisen von NGAL umfasst.

5. Verfahren nach Anspruch 1 zur weiteren Überwachung der Wirksamkeit einer Behandlung für eine Nierentubuluszellenverletzung, umfassend die folgenden Schritte:
3) Herstellen des Kontakts mindestens einer Urinprobe nach der Behandlung von dem menschlichen Patienten, der die Nierentubuluszellenverletzung erlitten hat und davor eine Behandlung erhalten hat, mit einem Fängerantikörper für NGAL, um die Bildung eines Komplexes aus dem Antikörper und NGAL zu ermöglichen; und
4) Nachweisen des Antikörper-NGAL-Komplexes in der Urinprobe nach der Behandlung.

6. Verfahren nach Anspruch 5, wobei der Schritt des Nachweisens des Antikörper-NGAL-Komplexes die folgenden Schritte umfasst:
(4i) Abtrennen von ungebundenem Material aus der Urinprobe von dem Fängerantikörper-NGAL-Komplex;
(4ii) Herstellen des Kontakts des Fängerantikörper-NGAL-Komplexes mit einem zweiten Antikörper zum Nachweisen von NGAL,
um die Bildung eines Komplexes zwischen NGAL und dem zweiten Antikörper zu ermöglichen;
(4iii) Abtrennen von ungebundenem zweitem Antikörper von dem Komplex aus NGAL und dem zweiten Antikörper; und
(4iv) Nachweisen des zweiten Antikörpers aus dem Komplex zwischen NGAL und dem zweiten Antikörper.

7. Verfahren nach Anspruch 5, wobei der Schritt 3) den Schritt des Herstellens des Kontakts der Urinprobe mit einem Medium, an dem der Fängerantikörper fixiert ist, umfasst.

8. Verwendung eines Kits, umfassend:
1) ein Mittel zum Gewinnen einer Menge einer Urinprobe;
2) ein Medium, an dem ein Fängerantikörper fixiert ist, der in der Lage ist, mit NGAL einen Komplex zu bilden; und
3) einen Test zum Nachweis eines Komplexes aus NGAL und dem Fängerantikörper in einem Verfahren nach Anspruch 1.

9. Verwendung nach Anspruch 8, wobei die Menge der Urinprobe geringer als 1 ml ist, besonders typisch geringer als 10 Mikroliter.

10. Verwendung nach Anspruch 8, wobei das Gewinnungsmittel ein Instrument umfasst, das Instrument eine Oberfläche umfasst, wobei die Oberfläche das Medium umfasst.

11. Verwendung nach Anspruch 8, wobei das Gewinnungsmittel einen Behälter zum Aufnehmen der Urinprobe umfasst, wobei die den Urin berührende Oberfläche des Behälters das Medium umfasst.

12. Verwendung nach Anspruch 8, wobei der Test einen ELISA umfasst.

13. Verwendung nach Anspruch 8, wobei das Gewinnungsmittel ein Instrument umfasst, das eine Kassette, die das Medium enthält, umfasst.

14. Verwendung nach Anspruch 8, wobei das Kit ein Kit zur Verwendung am Behandlungsort ist.

15. Verwendung nach Anspruch 14, wobei das Mittel zum Gewinnen der Urinprobe für eine Menge der Urinprobe von weniger als 1 ml, besonders typisch weniger als 10 Mikroliter ausgestaltet ist.

16. Verwendung eines Kits zur Verwendung am Behandlungsort nach Anspruch 15, wobei das Gewinnungsmittel ein Instrument umfasst, das ein Messstäbchen umfasst, wobei die Oberfläche des Messstäbchens das Medium umfasst.

17. Verwendung des Kits zur Verwendung am Behandlungsort nach Anspruch 15, wobei der Test einen farbmetrischen Messstäbchen-Test umfasst.

18. Verwendung eines Kits für einen kompetitiven enzymgekoppelten Immunadsorptionstest (ELISA), das einen für NGAL spezifischen Antikörper umfasst, um sein Vorliegen in einer Urinprobe des Menschen in einem Verfahren nach Anspruch 1 nachzuweisen.

19. Verwendung nach Anspruch 18 zum Untersuchen einer Urinprobe, die eine Flüssigkeitsmenge von etwa 1 Milliliter oder weniger umfassen kann.

20. Verfahren zum Identifizieren des Ausmaßes einer Nierentubuluszellenverletzung, die durch ein Ereignis verursacht wurde, wobei das Ereignis aus der Gruppe ausgewählt ist, die aus verminderter Blutzufuhr zu den Nieren, beeinträchtigter Herzfunktion, operativen Verfahren, Patienten auf Intensivstationen und der Verabreichung von Pharmazeutika, Radiokontrastmitteln oder anderen Arzneimittelsubstanzen an einen menschlichen Patienten besteht, wobei das Verfahren die folgenden Schritte umfasst:
1) Nachweisen in mindestens einer Urinprobe von einem menschlichen Patienten des Vorliegens eines Biomarkers für eine Nierentubuluszellenverletzung, wobei der Biomarker aus NGAL besteht; und
2) Feststellen des Ausmaßes Nierentubuluszellenverletzung basierend auf der Zeit des Beginns des Vorliegens des NGAL in der Urinprobe relativ zur Zeit des Ereignisses.

21. Verfahren nach Anspruch 1, wobei der Schritt 1) das Herstellen des Kontakts einer Urinprobe, die bis zu 1 Milliliter Urin von einem menschlichen Patienten umfasst, mit dem Antikörper für NGAL, um die Bildung eines Komplexes aus dem Antikörper und NGAL zu ermöglichen, umfasst.

## Revendications

1. Procédé de détection d'une lésion de cellules tubulaires rénales chez un patient humain causée par un événement, ledit événement étant choisi dans le groupe constitué de la diminution de l'apport sanguin aux reins, d'une insuffisance cardiaque, d'interventions chirurgicales, de patients en unités de soins intensifs et de l'administration de produits pharmaceutiques, de colorants de contraste radiologique ou d'autres substances médicamenteuses à un patient humain, ledit procédé comprenant les étapes de :
1) mise en contact d'un échantillon d'urine obtenu auprès d'un patient humain avec un anticorps pour un biomarqueur d'une lésion de cellules tubulaires rénales, le biomarqueur consistant en NGAL, pour permettre la formation d'un complexe de l'anticorps et du NGAL ; et
2) détection du complexe anticorps-NGAL.

2. Procédé selon la revendication 1, dans lequel une pluralité d'échantillons d'urine du sujet est obtenue par intermittence.

3. Procédé selon la revendication 2, dans lequel les échantillons d'urine sont obtenus en continu.

4. Procédé selon la revendication 1, dans lequel l'étape de détection du complexe anticorps-NGAL comprend la mise en contact du complexe avec un second anticorps pour détecter le NGAL.

5. Procédé selon la revendication 1, pour suivre en outre l'efficacité d'un traitement pour une lésion de cellules tubulaires rénales, comprenant les étapes de :
3) mise en contact d'au moins un échantillon d'urine post-traitement provenant du patient humain ayant subi la lésion de cellules tubulaires rénales et recevant un traitement pour celle-ci, avec un anticorps de capture pour le NGAL afin de permettre la formation d'un complexe de l'anticorps et du NGAL ; et
4) détection du complexe anticorps-NGAL dans l'échantillon d'urine post-traitement.

6. Procédé selon la revendication 5, dans lequel l'étape de détection du complexe anticorps-NGAL comprend les étapes de :
(4i) séparation de tout matériau non lié de l'échantillon d'urine du complexe anticorps de capture-NGAL ;
(4ii) mise en contact du complexe anticorps de capture-NGAL avec un second anticorps pour détecter le NGAL, afin de permettre la formation d'un complexe entre le NGAL et le second anticorps ;
(4iii) séparation de tout second anticorps non lié du complexe NGAL-second anticorps ; et
(4iv) détection du second anticorps du complexe NGAL-second anticorps.

7. Procédé selon la revendication 5, dans lequel l'étape 3) comprend l'étape de mise en contact de l'échantillon d'urine avec un milieu sur lequel est fixé l'anticorps de capture.

8. Utilisation d'une trousse comprenant :
1) un moyen pour acquérir une quantité d'un échantillon d'urine ;
2) un milieu sur lequel est fixé un anticorps de capture capable de se complexer avec le NGAL ; et
3) un essai pour la détection d'un complexe de NGAL et de l'anticorps de capture, dans un procédé selon la revendication 1.

9. Utilisation selon la revendication 8, dans laquelle la quantité d'échantillon d'urine est inférieure à 1 ml, plus typiquement inférieure à 10 microlitres.

10. Utilisation selon la revendication 8, dans laquelle le moyen d'acquisition comprend un instrument comprenant une surface, la surface comprenant le milieu.

11. Utilisation selon la revendication 8, dans laquelle le moyen d'acquisition comprend un récipient pour accepter l'échantillon d'urine, dans laquelle la surface du récipient en contact avec l'urine comprend le milieu.

12. Utilisation selon la revendication 8, dans laquelle l'essai comprend un ELISA.

13. Utilisation selon la revendication 8, dans laquelle le moyen d'acquisition comprend un instrument comprenant une cassette contenant le milieu.

14. Utilisation selon la revendication 8, dans laquelle la trousse est une trousse pour point de traitement.

15. Utilisation selon la revendication 14, dans laquelle le moyen pour acquérir l'échantillon d'urine est conçu pour une quantité de l'échantillon d'urine inférieure à 1 ml, plus typiquement inférieure à 10 microlitres.

16. Utilisation d'une trousse pour point de traitement selon la revendication 15, dans laquelle le moyen d'acquisition comprend un instrument comprenant un bâton-immersion, la surface du bâton-immersion comprenant le milieu.

17. Utilisation de la trousse pour point de traitement selon la revendication 15, dans laquelle l'essai comprend un essai colorimétrique au bâton-immersion.

18. Utilisation d'une trousse d'essai par immunosorbant lié à une enzyme compétitif (ELISA) comprenant un anticorps spécifique au NGAL pour détecter sa présence dans un échantillon d'urine de l'être humain dans un procédé selon la revendication 1.

19. Utilisation selon la revendication 18 pour le dosage d'un échantillon d'urine qui peut comprendre une quantité de fluide d'environ 1 millilitre ou moins.

20. Procédé d'identification de l'étendue d'une lésion de cellules tubulaires rénales, causée par un événement, ledit événement étant choisi dans le groupe constitué de la diminution de l'apport sanguin aux reins, d'une insuffisance cardiaque, d'interventions chirurgicales, de patients en unités de soins intensifs et de l'administration de produits pharmaceutiques, de colorants de contraste radiologique ou d'autres substances médicamenteuses à un patient humain, ledit procédé comprenant les étapes de
1) détection dans au moins un échantillon d'urine provenant d'un patient humain de la présence d'un biomarqueur pour une lésion de cellules tubulaires rénales, ledit biomarqueur consistant en NGAL ; et
2) détermination de l'étendue de la lésion de cellules tubulaires rénales sur la base du moment où le NGAL est apparu dans l'échantillon d'urine, par rapport au moment de l'événement.

21. Procédé selon la revendication 1, dans lequel l'étape 1) comprend la mise en contact d'un échantillon d'urine comprenant jusqu'à 1 millilitre d'urine d'un patient humain avec l'anticorps pour le NGAL, afin de permettre la formation d'un complexe de l'anticorps et du NGAL.
